(19) Europäisches Patentamt
European Patent Office
Office européen des brevets

(11) Veröffentlichungsnummer : **0 094 951**
**B1**

(12) # EUROPÄISCHE PATENTSCHRIFT

(45) Veröffentlichungstag der Patentschrift : **30.10.85**

(21) Anmeldenummer : **82903452.9**

(22) Anmeldetag : **25.11.82**

(86) Internationale Anmeldenummer : **PCT/DE 82/00222**

(87) Internationale Veröffentlichungsnummer : **WO/8301951 (09.06.83 Gazette 83/14)**

(51) Int. Cl.⁴ : **C 07 D307/935**, C 07 D407/12, C 07 F 7/18, A 61 K 31/34, A 61 K 31/557// C07F9/40, C07C69/65, C07C69/593, C07C69/533, C07C57/52

(54) **NEUE PROSTACYCLINE UND VERFAHREN ZU IHRER HERSTELLUNG.**

(30) Priorität : **27.11.81 DE 3147714**

(43) Veröffentlichungstag der Anmeldung : **30.11.83 Patentblatt 83/48**

(45) Bekanntmachung des Hinweises auf die Patenterteilung : **30.10.85 Patentblatt 85/44**

(84) Benannte Vertragsstaaten : **AT BE CH DE FR GB LI LU NL SE**

(56) Entgegenhaltungen :
**Angewandte Chemie, Band 15, Br. 11, (Weinheim, DE) H.D. Scharf u. a. " Synthesis of furan derivatives from 4-benzoyloxy-1,3-dioxolane derivatives" Seite 682**
**Journal of the American Chemical Society, Band 100, Nr. 2, 18. Januar 1978 (Columbus, Ohio, US) "Concerning the chemical identity on 6,9alpha-oxido-11alpha, 15alpha-dihydroxyprosta-7(8), (E)13-dienoic acid", Seiten 643-645**
**Tetrahedron Letters, Nr. 32, August 1979 (Oxford, GB) K. Ohno u. a. "-4,5,6,7-tetrahydro-PGI1, a stable and potent inhibitor of blood platelet aggregation" Seiten 3003, 3004**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **NICKOLSON, Robert**
**Fuchsienweg 12c**
**D-1000 Berlin 47 (DE)**
Erfinder : **VORBRUGGEN, Helmut**
**Wilkestrasse 7**
**D-1000 Berlin 27 (DE)**
Erfinder : **CASALS-STENZEL, Jorge**
**Sertoriusring 295**
**D-6500 Mainz 21 (DE)**
Erfinder : **HABEREY, Martin**
**Neckarsulmer Strasse 15**
**D-1000 Berlin 46 (DE)**

**EP 0 094 951 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Prostacyclin-Derivate, Verfahren zu ihrer Herstellung sowie ihre Verwendung als Arzneimittel.

Prostacyclin ($PGI_2$), einer der Hauptfaktoren bei der Blutplättchenaggregation, wirkt dilatierend auf verschiedene Blutgefäße (Science *196*, 1072) und könnte daher als Mittel zur Blutdrucksenkung in Betracht kommen. $PGI_2$ besitzt jedoch nicht die für ein Arzneimittel notwendige Stabilität. So beträgt seine Halbwertszeit bei physiologischen pH-Werten und bei Raumtemperatur nur wenige Minuten.

Es wurde gefunden, daß die Aromatisierung des Tetrahydrofuranringes im Prostacyclin zu einer Stabilisierung des Prostacyclinmoleküls führt, wobei das pharmakologische Wirkungsspektrum erhalten bleibt und die Wirkungsdauer der neuen Prostacycline deutlich verlängert wird.

Die erfindungsgemäßen Verbindungen wirken blutdrucksenkend und bronchodilatatorisch. Sie sind außerdem zur Inhibierung der Thrombozytenaggregation, der Vasodilatation und der Magensäuresekretion geeignet.

Die Erfindung betrifft Prostacycline der allgemeinen Formel I

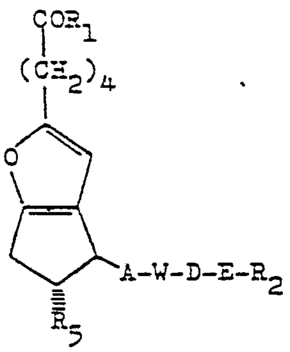

(I)

worin

$R_1$ den Rest $OR_3$, wobei $R_3$ Wasserstoff oder gegebenenfalls durch Halogen, Aryl, $C_1$-$C_4$-Alkoxy oder $C_1$-$C_4$-Dialkylamino substituiertes Alkyl mit 1-10 C-Atomen, Cycloalkyl, Aryl oder einen heterocyclischen Rest bedeuten kann, oder den Rest $NHR_4$ mit $R_4$ in der Bedeutung eines Wasserstoff, Alkanoyl- oder Alkansulfonylrestes mit je 1-10 C-Atomen darstellt,

A eine $-CH_2-CH_2-$ oder trans $-CH=CH$-Gruppe,

W eine 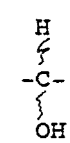 oder eine 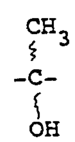 Gruppe,

in denen die OH-Gruppe jeweils mit einem Benzoyl oder Alkansäurerest mit 1-4 C-Atomen verestert oder mit einem Tetrahydropyranyl-, Tetrahydrofuranyl-, $C_1$-$C_4$-Alkoxyalkyl- oder Tri-($C_1$-$C_4$-alkyl)-silylrest veräthert sein kann, wobei die freie oder veresterte OH-Gruppe α- oder β-ständig sein kann,

D und E gemeinsam eine direkte Bindung oder

D eine geradkettige oder verzweigte Alkylengruppe mit 1-5 C-Atomen,

E ein Sauerstoffatom oder eine direkte Bindung,

$R_2$ eine gerad- oder verzweigtkettige Alkylgruppe mit 1-6 C-Atomen, eine gerad- oder verzweigtkettige Alkenyl-gruppe mit 2-6 C-Atomen, die durch Phenyl, Halogen oder $C_1$-$C_4$-Alkyl substituiert sein kann und wenn D und E gemeinsam eine Direktbindung bedeuten, einen gegebenenfalls in 1-Stellung durch Halogen oder $C_1$-$C_4$-Alkyl substituierten Alkinylrest mit 2-6 C-Atomen, oder, wenn $R_1$ eine Hydroxygruppe, $R_5$ eine Hydroxygruppe, A eine trans $-CH=CH$-Gruppe,

W eine 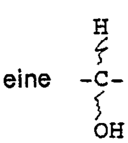 Gruppe,

D eine $-CH_2$-Gruppe und E ein O-Atom bedeuten, Phenyl,

$R_5$ eine Hydroxygruppe, die mit einem Alkansäurerest mit 1-4 C-Atomen verestert oder mit einem Tetrahydropyranyl-, Tetrahydrofuranyl-, Alkoxyalkyl- oder Trialkylsilylrest veräthert sein kann und, falls $R_3$ Wasserstoff darstellt, deren Salze mit physiologisch verträglichen Basen bedeuteten.

Als Alkylgruppe $R_3$ sind gerade oder verzweigte Alkylgruppen mit 1-10 C-Atomen zu betrachten, wie

beispielsweise Methyl, Äthyl, Propyl, Butyl, Isobutyl, tert.-Butyl, Pentyl, Neopentyl, Heptyl, Hexyl, Decyl. Die Alkylgruppen $R_3$ können gegebenenfalls 1 bis mehrfach substituiert sein durch Halogenatome, Alkoxygruppen mit 1-4 C-Atomen, gegebenenfalls substituierte Arylgruppen, Dialkylamine und Trialkylammonium mit 1-4 C-Atomen. Bevorzugt sind solche Alkylgruppen, die einfach substituiert sind. Als Substituenten seien beispielsweise genannt Fluor-, Chlor- oder Bromatome, Phenyl, Dimethylamin, Methoxy, Äthoxy. Als bevorzugte Alkylgruppen $R_3$ sind solche mit 1-4 C-Atomen, wie z. B. Methyl, Äthyl, Propyl, Dimethylaminopropyl, Isobutyl und Butyl zu nennen.

Als Arylgruppen $R_3$ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Cycloalkylgruppe $R_3$ kann im Ring 4-10, vorzugsweise 4 bis 6 Kohlenstoffatome enthalten. Die Ringe können durch Alkylgruppen mit 1-4 Kohlenstoffatomen substituiert sein. Beispielsweise seien genannt Cyclobutyl, Cyclopentyl, Cyclohexyl, Methylcyclohexyl und Adamantyl.

Als heterocyclische Gruppen $R_3$ kommen 5- und 6-gliedrige Heterocyclen in Frage, von denen diejenigen mit einem Heteroatom, wie z. B. Stickstoff, Sauerstoff oder Schwefel besonders bevorzugt sind. Beispielsweise seien genannt: 2-Furyl, 2-Thienyl, 3-Thienyl, 2-Pyridyl, 3-Pyridyl, 4-Pyridyl u. a.

Als Säurerest $R_4$ kommen physiologisch verträgliche Säurereste in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien Alkyl-, Hydroxy-, Alkoxy-, Oxo- oder Aminogruppen oder Halogenatome erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielsweise Alkansulfonsäuren mit 1-10 C-Atomen wie z. B. Methansulfonsäure, Äthansulfonsäure, Isopropansulfonsäure, β-Chloräthansulfonsäure, Butansulfonsäure, Cyclopentansulfonsäure, Cyclohexan sulfonsäure aber auch Benzolsulfonsäure, p-Toluolsulfonsäure, p-Chlor-benzolsulfonsäure, N,N-Dimethylaminosulfonsäure, N,N-Di-äthylaminosulfonsäure, N,N-Bis-(β-chloräthyl)-aminosulfonsäure, N,N-Diisobutylaminosulfonsäure, N,N-Dibutylamino-sulfonsäure, Pyrrolidino-, Piperidino-, Piperazino-, N-Methylpiperazino- und Morpholinosulfonsäure in Frage.

Die Hydroxygruppen $R_5$ und in W können funktionell abgewandelt sein, beispielsweise durch Verätherung oder Veresterung, wobei die freien oder abgewandelten Hydroxygruppen in W α- oder β-ständig sein können, und wobei freie Hydroxygruppen bevorzugt sind. Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethyl-silyl-, Dimethyl-tert.-butylsilyl- und Tribenzyl-silylrest. Als Acylreste kommen $C_1$-$C_4$-Alkanoylreste wie beispielsweise Acetyl, Propionyl, Butyryl oder Benzoyl in Frage.

Als Alkyl- und Alkenylgruppen $R_2$ kommen gerad- und verzweigtkettige Alkyl- mit 1-10 und Alkenylreste mit 2-10, insbesondere 1-6 bzw. 2-6 C-Atomen, in Frage, die gegebenenfalls durch gegebenenfalls substituiertes Phenyl, Alkyl mit 1-4 C-Atomen oder Halogen substituiert sein können. Beispielsweise genannt seien Methyl, Athyl, Propyl, Butyl, Isobutyl, tert.-Butyl, Pentyl, Hexyl, Heptyl, Octyl, Butenyl, Isobutenyl, Propenyl, Pentenyl, Hexenyl sowie Benzyl, und für den Fall, daß D und E gemeinsam eine Direktbindung bedeuten, gegebenenfalls in 1-Stellung durch Fluor oder $C_1$-$C_4$-Alkyl substituiertes Alkinyl mit 2-6 C-Atomen. Als Alkinylreste kommen in Betracht: Äthinyl, Propin-1-yl, Propin-2-yl, 1-Methylpropin-2-yl, 1-Fluorpropin-2-yl, 1-Äthylpropin-2-yl, 1-Fluorbutin-2-yl, Butin-2-yl, Butin-3-yl, 1-Methyl-butin-3-yl, 1-Methylpentin-3-yl, 1-Fluor-pentin-3-yl, 1-Methyl-pentin-2-yl, 1-Fluorpentin-2-yl, 1-Methylpentin-4-yl, 1-Fluorpentin-4-yl, Hexin-1-yl, 1-Methylhexin-2-yl, 1-Fluorhexin-2-yl, 1-Methylhexin-3-yl, 1-Methylhexin-4-yl, Hexin-3-yl, 1,1-Dimethylpropin-2-yl, 1,1-Dimethylbutin-3-yl, 1,1-Dimethyl-pentin-3-yl, 1,1-Dimethylpentin-4-yl, 1,1-Dimethyl-hexin-3-yl, 1,1-Dimethylhexin-4-yl usw. Für Halogen als Substituent der Alkyl- und Alkenylgruppen $R_2$ kommen Brom, Chlor und Fluor in Betracht. Bevorzugt sind Chlor und Fluor.

Als Alkylengruppe D kommen geradkettige oder verzweigtkettige Alkylenreste, die eine Doppelbindung enthalten können, vorzugsweise jedoch gesättigte mit 1-10, insbesondere 1-5 C-Atomen, in Frage, die gegebenenfalls durch Fluoratome oder $C_1$-$C_4$-Alkyl besonders in 1- oder 2-Stellung

substituiert sein können. Beispielsweise seien genannt : methylen, fluormethylen, äthylen, 1,2-propylen, äthyläthylen, trimethylen, tetramethylen, pentamethylen, 1-methyl-tetramethylen, 1-methyl-trimethylen, 2-methyl-trimethylen, 2-methyl-tetramethylen, 1,1-Trimethylen-äthylen, 1,2-Methylenäthylen. Wenn eine Doppelbindung vorliegt, befindet sie sich in den Alkylenresten mit 4-10 C-Atomen in 2-, 3- oder 4-Stellung.

Zur Salzbildung mit den freien Säuren ($R_3 = H$) sind anorganische und organische Basen geeignet, wie sie dem Fachmann zur Bildung physiologisch verträglicher Salze bekannt sind.

Beispielsweise seien genannt : Alkalihydroxide, wie Natrium- und Kaliumhydroxid, Erdalkalihydroxide, wie Calciumhydroxid, Ammoniak, Amine, wie Äthanolamin, Diäthanolamin, Triäthanol-amin, N-Methylglucamin, Morpholin, Tris(hydroxymethyl)-methylamin usw.

Die Erfindung betrifft ferner ein Verfahren zur Herstellung der erfindungsgemäßen Prostacycline der allgemeinen Formel I, dadurch gekennzeichnet, daß man eine Verbindung der allgemeinen Formel II

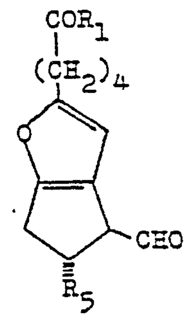

(II)

worin $R_1$ und $R_5$ die oben angegebene Bedeutungen aufweisen mit einem Alkalisalz des Phosphonats der allgemeinen Formel III

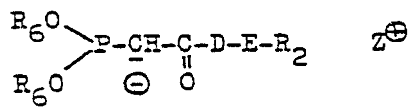

(III)

worin D, E und $R_2$ die oben angegebenen Bedeutungen haben und $R_6$ eine $C_1$-$C_4$-Alkyl und Z die Alkalimetalle Li, Na oder K bedeuten, umsetzt und je nach der gewünschten Bedeutung reduziert oder mit Methylmagnesiumbromid oder mit Methyl-Lithium umsetzt oder gegebenenfalls in die Stereoisomeren trennt oder den 1-Ester verseift oder die 1-Säure verestert oder mit einem Isocyanat der Formel $R_4$—N=C=O umsetzt, worin $R_4$ die bereits angegebene Bedeutung hat, oder OH-Schutzgruppen abspaltet.

Die Umsetzung der Verbindung der allgemeinen Formel II mit Verbindungen der Formel III wird bei Temperaturen von − 50 bis 50 °C, vorzugsweise bei − 30 bis 20 °C, in einem organischen Lösungsmittel, vorzugsweise Dimethoxyäthan in 0,5-10 Std. unter Inertgas (wie z. B. $N_2$ oder Ar) und unter Rühren vorgenommen.

Die Verseifung der Prostacyclinester wird nach den dem Fachmann bekannten Methoden durchgeführt, beispielsweise mit basischen Katalysatoren. Die Einführung der Estergruppe, bei welcher $R_3$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. *8*, Seiten 389-394 (1954)].

Die Einführung der Estergruppe $OR_3$ für $R_1$, bei welcher $R_3$ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Esssigester, Tetrahydrofuran, vorzugsweise Chloroform, in Frage. Die Reaktion wird bei Temperaturen zwischen − 30 °C und + 50 °C, vorzugsweise bei + 10 °C, durchgeführt.

Die Prostacyclin-Derivate der allgemeinen Formel I mit $R_3$ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird das Prostacyclin der Formel I zum Beispiel in einem geeigneten Lösungsmittel, beispielsweise Äthanol, Aceton, Acetonitril, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid, Benzol oder Chloroform unter Verwendung eines sauren Katalysators, wie zum Beispiel $POCl_3$, p-Toluol-sulfonsäure oder wasserfreier Mineralsäuren umgesetzt. Das Dihydropyran wird im Überschuß angewandt, vorzugsweise in der 2- bis 10-fachen Menge des theoretischen Bedarfs. Die Umsetzung ist normalerweise bei 0 ° C bis 30 °C nach 15-30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u. a., in Gegenwart einer tertiären Aminbase, wie z. B. Pyridin, Dimethylaminopyridin etc. umsetzt.

Die Freisetzung einer funktionell abgewandelten OH-Gruppe zu den Verbindungen der allgemeinen Formel I erfolgt nach bekannten Methoden. Beispielsweise wird die Abspaltung der Ätherschutzgruppen in einer wässrigen Lösung einer organischen Säure, wie zum Beispiel Essigsäure, Propionsäure u. a. oder in einer wässrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid oder mit KF in Gegenwart eines Kronenäthers. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignete Calciumcarbonat, Calciumhydroxyd und Bariumcarbonat. Die Umsetzung erfolgt bei − 10 °C bis 70 °C, vorzugsweise bei 25 °C.

Die Umsetzung der Verbindung der allgemeinen Formel I mit $R_3$ in der Bedeutung eines Wasserstoffatoms mit einem Isocyanat der allgemeinen Formel

$$R_4{-}N{=}C{=}O$$

worin $R_4$ die obenangegebene Bedeutung hat, erfolgt gegebenenfalls unter Zusatz eines tertiären Amins, wie zum Beispiel Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Benzol, Toluol, Dimethylsulfoxid, bei Temperaturen ober- oder unterhalb Raumtemperatur, zum Beispiel zwischen − 80 °C bis 100 °C, vorzugsweise bei 0 °C bis 30 °C, vorgenommen werden.

Die als Ausgangsmaterial dienenden Verbindungen der allgemeinen Formel II können analog, wie in die Beispielen 1-14 beschrieben wird, hergestellt werden. Analog Beispiel 4 und 6 lassen sich die entsprechenden Schwefelverbindungen herstellen und in analoger Reaktion zu der Verbindung in Beispiel 5 bzw. zu der entsprechenden Schwefelverbindung des Beispiels 7 umsetzen.

Die Oxidation der 4-Hydroxymethylgruppe in den Verbindungen der Formel IV

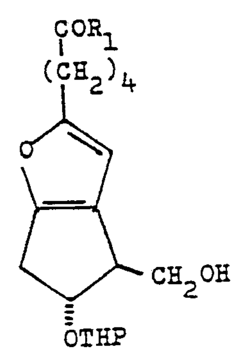

(IV)

worin $R_1$ die angegebene Bedeutung hat und THP einen Tetrahydropyranyl-Rest darstellt, zu den

Aldehyden der allgemeinen Formel V

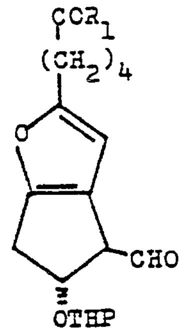

(V)

worin $R_1$ und THP die oben angegebenen Bedeutungen haben, wird nach den dem Fachmann bekannten Methoden vorgenommen. Als Oxydationsmittel oder -methoden können beispielsweise dienen : Collins-Reagenz (Tetrahedron Letters, 1968, 3368), Jones-Reagenz (J. Chem. Soc. 1953, 2555), Pyridiniumchlorochromat (Tetrahedron Letters, 1975, 2647, Pyridinium-dichromat (Tetrahedron Letters, 1979, 399) oder die Moffat-Pfitzner-Methode. Die Oxidation wird mit Collins-Reagenz bei − 20 °C bis + 30 °C, vorzugsweise 0-5 °C oder mit Jones-Reagenz bei − 40 °C bis + 30 °C, vorzugsweise − 30 °C bis 0 °C oder mit Pyridiniumchlorochromat oder Pyridiniumdichromat bei − 20 °C bis + 40 °C, vorzugsweise bei 20 °C − 30 °C in einem gegenüber Oxydationsmittel inerten Lösungsmittel durchgeführt. Als Lösungsmittel können je nach dem verwendeten Reagenz Methylenchlorid, Chloroform, Äthylenchlorid, Dimethylformamid, Pyridin u. a. verwendet werden.

Werden letztlich Endprodukte gewünscht, die frei Hydroxylgruppen enthalten, geht man zweckmäßigerweise von Ausgangsprodukten aus, in denen diese durch vorzugsweise leicht abspaltbare Äther- oder Acylreste intermediär geschützt sind.

Die Verbindungen dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Prostaglandin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken ; Behandlung von Schlaganfall, Prophylaxe und Therapie koronarer Herzerkrankungen, koronarer Thrombose, des Herinfarkts, peripherer Arterienerkrankungen, Arteriosklerose und Thrombose, Prophylaxe und Therapie ischämischer Attacken des ZNS-Systems, Therapie des Schocks, Inhibierung der Bronchokonstriktion, Inhibierung der Magensäuresekretion und Zytoprotektion der Magen- und Darmschleimhaut sowie Zytoprotektion an der Leber und Bauchspeicheldrüse ; antiallergische Eigenschaften, Senkung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc... Außerdem besitzen die neuen Prostaglandinanaloga antiproliferative Eigenschaften. Die neuen Prostacycline können auch in Kombination z. B. mit β-Blockern oder Diuretika verwendet werden. Die Dosis der Verbindungen ist 1-1 500 µg/kg/Tag, wenn sie am menschlichen Patienten verabreicht werden. Die einheitsdosis für den pharmazeutisch akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, z. B. zur Herstellung von Blutdrucksenkern dienen.

Die vorliegende Erfindung betrifft auch Prostacyclinzwischenprodukte der allgemeinen Formel VI

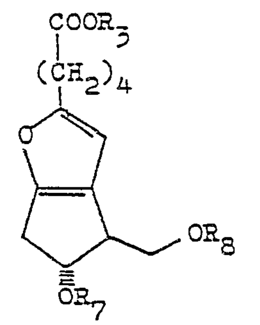

(VI)

worin $R_3$ die oben angegebene Bedeutung hat und $R_7$ und $R_8$ die für $R_5$ angegebenen Schutzgruppen, eine Tri-($C_1$-$C_4$)-alkylsilylgruppe oder Benzylgruppe bedeuten und ein Verfahren zur Herstellung von Verbindungen der Formel VI

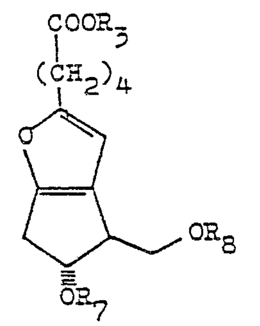

(VI)

worin $R_3$, $R_7$ und $R_8$ die oben angegebenen Bedeutungen haben, dadurch gekennzeichnet, daß man Verbindungen der allgemeinen Formel VII

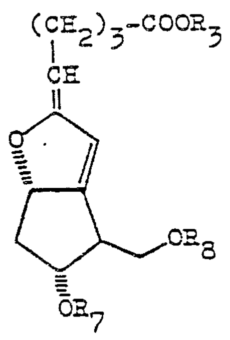

(VII)

mit $R_3$, $R_7$ und $R_8$ in den angegebenen Bedeutungen bei erhöhter Temperatur mit schwachen Lewissäuren behandelt.

Von Prostacyclin weiß man, daß es innerhalb von Minuten durch Umwandlung in das entsprechende 6-Ketoprostaglandin seine pharmakologische Aktivität verliert. (S. Moncada and J.R. Vane in Biochemical Aspects of Prostaglandins and Thromboxanes, N. Kharasch and J. Fried (editors), Academic Press, New York, 1977, page 155 ff.) Die Dienole der Formel VII stellen ebenfalls relativ instabile Verbindungen dar und zersetzen sich bereits unter Einwirkung von Luft und Spuren von Wasser. Durch Erhitzen in Gegenwart schwacher Lewissäuren wie z. B. Magnesiumsulfat, Borsäure, $(NH_4)_2SO_4$, $NH_4Cl$, Kieselgel, $Al_2O_3$, Al-tris (isopropylat), oder schwache organische Säuren können die Dienole VII überraschend in die stabilen Furanprostacycline der Formel VI umgewandelt werden.

Die Aromatisierung wird in trockenen inerten organischen aprotischen Lösungsmitteln, wie Benzol, Toluol, Xylol, Tetrahydrofuran, Dioxan, Diglyme, Äther, Hexamethyldisilazan oder Trimethylsilyldiäthylamin bei 30-200 °C, bevorzugt bei 50-130° durchgeführt.

Beispiel 1

(1S,5R,6S,7R)-6-tert.-Butyldimethylsilyloxymethyl-7-benzoyloxy-2-oxabicyclo [3.3.0] octan-3-on

Eine Lösung von 11.052 g (1S, 5R, 6S, 7R)-6-Hydroxymethyl-7-benzoyloxy-2-oxabicyclo [3.3.0] octan-3-on (DE-OS 26 10 718) in 54.0 ml abs. Dimethylformamid wird mit 3.03 g Imidazol und 6.63 g tert.-Butyldimethylsilylchlorid versetzt und das Gemisch 3 Stunden bei Raumtemperatur gerührt. Die Lösung wird anschließend in 500 ml Eiswasser gefällt, der Niederschlag abgesaugt und der Rückstand in Methylenchlorid gelöst. Die organische Lösung wird über Magnesiumsulfat getrocknet und eingeengt. Das Rohprodukt wird aus Hexan umkristallisiert. Man erhält 15.30 g der Titelverbindung, Schmp. 75.8 °C.

Beispiel 2

(1S,5R,6S,7R)-6-tert.-Butyldimethylsilyloxymethyl-7-hydroxy-2-oxabicyclo [3.3.0] octan-3-on

Zu einer Lösung von 11.716 g des im Beispiels 1 hergestellten Silylethers in 122 ml abs. MeOH werden 1.845 g wasserfreies Kaliumcarbonat eingetragen und das Gemisch 3 Stunden unter Argon gerührt. Die Reaktionslösung wird anschliessend im Eisbad gekühlt und vorsichtig mit 1.86 ml konz. Salzsäure tropfenweise versetzt. Überschüssiges Methanol wird weitgehend bei Raumtemperatur im Vakuum eingeengt, der Rückstand mit 300 ml Methylenchlorid versetzt und die Lösung über Magnesiumsulfat getrocknet, abfiltriert und eingeengt. Der ölige Rückstand wird durch Verreiben mit Hexan zur Kristallisation gebracht. Man erhält 6.562 g der Titelverbindung, Schmp. 60.7 °C.

Nach Chromatographie der Mutterlaugen über 100 g Kieselgel in einem Methylenchlorid/25 % Aceton-Methylenchlorid-Gradienten und Kristallisation aus Hexan, werden weitere 1.089 g der Titelverbindung, Schmp. 62.3 °C erhalten.

Beispiel 3

(1S, 5R, 6S, 7R)-6-tert.-Butyldimethylsilyloxymethyl-7-(tetrahydropyran-2-yloxy)-2-oxabicyclo [3.3.0] octan-3-on

Eine Lösung von 9.608 g des im Beispiel 2 beschriebenen Diols in 134 ml abs. Methylenchlorid wird mit 4.08 ml 2.3-Dihydro-lll-pyran und 56.3 mg p-Toluolsulfonsäure versetzt. Nach 20 Minuten wird die Reaktion durch Zugabe von 0.06 ml Triethylamin beendet, überschüssiges Methylenchlorid durch Destillation im Vakuum bei Raumtemperatur entfernt und der Rückstand auf 200 g Kieselgel in einem Hexan/25 % Essigester-Hexan-Gradienten chromatographiert. Es werden 13.391 g der Titelverbindung als Öl erhalten.

IR : 2 940, 1 760, 1 450, 1 340, 1 245, 1 160, 1 110, 1 060, 1 030, 970, 835, 775 $cm^{-1}$.

Beispiel 4

(1S,4S,5R,6S,7R)-4-Phenylselenyl-6-tert.-butyldimethylsilyloxymethyl-7-(tetrahydropyran-2-yloxy)-2-oxabicyclo [3.3.0] octan-3-on

Eine Lösung von Lithiumhexamethyldisilazid wird zubereitet, indem man 50 ml n-Butyllithium-Lösung (1.21 M in Hexan) zu einer auf − 10 °C gekühlten Lösung von 12,5 ml Hexamethyldisilazan in 100 ml abs. Tetrahydrofuran unter Argon zutropft. Eine auf − 70 °C gekühlte Lösung von 18.55 g des nach Beispiel 3 hergestellten Tetrahydropyranylethers in 400 ml abs. Tetrahydrofuran wird mit der frisch zubereiteten Lösung von Lithiumhexamethyldisilazid innerhalb von 10 Minuten nachgerührt. Zu diesem Gemisch wird eine Lösung von 11.45 g Phenylselenylchlorid in 105 ml abs. Tetrahydrofuran innerhalb 5 Minuten zugetropft und nach beendeter Zugabe lässt man die Lösung auf Raumtemperatur erwärmen. Die Lösung wird mit ca. 50 g Kieselgel versetzt, überschüssiges Lösungsmittel durch Destillation im Vakuum entfernt und der Rückstand durch Chromatographie auf Kieselgel in einem Hexan/15 % Essigester-Hexan-Gradienten aufgereinigt. Man erhält 15.41 g der Titelverbindung.

IR : 2 950, 1 760, 1 570, 1 450, 1 340, 1 240, 1 170, 1 110, 1 070, 1 030, 835, 775, 740, 690 $cm^{-1}$.

Beispiel 5

(1S, 6S, 7R)-6-tert.-Butyldimethylsilyloxymethyl-7-(tetrahydropyran-2-yloxy)-2-oxabicyclo [3.3.0] oct-4-en-3-on

Zu einer Lösung von 13.14 g des im Beispiels 4 hergestellten Phenylselenids in 375 ml Methylenchlorid wird ein Gemisch bestehend aus 81 ml 30 %igem Wasserstoffperoxid und 165 ml Wasser zugegeben und die Mischung wird 1 Stunde bei Raumtemperatur unter Argon gerührt. Nach 1 Stunde wird die Reaktionslösung mit Ether verdünnt, die wässrige Phase abgetrennt und die organische Phase mit Wasser gewaschen. Die Ether-Lösung wird über Magnesiumsulfat getrocknet und im Vakuum eingeengt. Das Rohprodukt wird über 200 g Kieselgel in einem Hexan/15 % Essigester-Hexan-Gradienten chromatographiert. Man erhält 8.66 g der Titelverbindung als Öl.

IR : 2 940, 1 760, 1 650, 1 450, 1 370, 1 330, 1 310, 1 250, 1 100, 1 030, 950, 835, 775 $cm^{-1}$.

Beispiel 6

(1S, 5S, 6S, 7R)-5-Phenylselenyl-6-tert.-butyldimethylsilyloxymethyl-7-(tetrahydropyran-2-yloxy)-2-oxabicyclo [3.3.0] octan-3-on

Zu einer auf 0 °C gekühlten Suspension von 1.698 g Natriumborhydrid in 41.6 ml abs. Ethanol werden portionsweise 6.45 g Diphenyldiselenid innerhalb von 30 Minuten eingetragen. Nach Beendigung der Gas-Entwicklung wird eine Lösung von 7.20 g des im Beispiel 5 hergestellten ungesättigten Lactons in 68 ml abs. Ethanol zur bereits hergestellten Lösung von Natriumselenophenolat zugegeben und das Gemisch 1.5 Stunden bei Raumtemperatur unter Argon gerührt. Die Lösung wird anschließend in 1.0 l gesättigte Natriumchlorid-Eis-Gemisch gegossen und die wäßrige Phase 4 mal mit 150 ml Portionen Ether ausgezogen. Die Ether-Lösung wird mit halbgesättigter Natriumchlorid-Lösung neutral gewaschen, über Magnesiumsulfat getrocknet und im Vakuum zur Trockne eingeengt. Das Rohprodukt wird über 500 g Kieselgel in einem Hexan/12 % Essigester-Hexan-Gradienten chromatographiert. Man erhält 6.265 g der Titelverbindung.

IR : 2 950, 1 760, 1 560 (schwach), 1 450, 1 430, 1 340, 1 250, 1 120, 1 090, 1 070, 970, 835, 775, 740, 690 $cm^{-1}$.

Beispiel 7

(1S,3RS,5S,6S,7R)-5-Phenylselenyl-6-tert.-butyldimethylsilyloxymethyl-7-(tetrahydropyran-2-yloxy)-2-oxabicyclo [3.3.0]-3-ol

Zu einer auf − 70 °C gekühlten Lösung von 5,64 g des im Beispiel 6 beschriebenen Lactons in 137 ml Toluol werden 19.3 ml Dibah-T-Lösung (20 % in Toluol) zugegeben und das Gemisch wird 0,5 Stunden unter Argon gerührt. Anschliessend wird überschüssiges Dibah durch vorsichtiges Zutropfen von 1.0 ml Isopropanol und 9.63 ml Wasser vernichtet und das Gemisch 3 Stunden bei Raumtemperatur weiter gerührt. Der weisse Niederschlag wird abgesaugt, der Rückstand mit Methylenchlorid gewaschen, die zusammengefassten organischen Phasen werden über Magnesiumsulfat getrocknet und anschliessend eingeengt. Das Rohprodukt wird über 100 g Kieselgel in einem Hexan/10 % Aceton-Hexan-Gradienten chromatographiert.

Man erhält 5.15 g der Titelverbindung.

Ir : 3 700, 2 940, 1 560, 1 460, 1 430, 1 340, 1 250, 1 080 (breit) 1 020, 835, 775, 735, 695 $cm^{-1}$.

Beispiel 8

(1S,2S,3S,4R)-2-[(EZ)-6-Methoxycarbonyl-2-hexenyl]-2-phenylselenyl-3-tert.-butyldimethylsilyloxymethyl-4-(tetrahydropyran-2-yloxy)-cyclopentan-1-ol

Eine auf − 10 °C gekühlte Lösung von 33.30 ml Hexamethyldisilazan in 110 ml abs. Tetrahydrofuran wird mit 144.35 ml Butyllithium-Lösung (1.11 M in Hexan) unter Argon versetzt. Die frisch zubereitete Lösung von Lithiumhexamethyldisilazid wird innerhalb von 5 Minuten zu einer Suspension 35.24 g Carboxybutyltriphenylphosphoniumbromid in 343 ml Tetrahydrofuran und das Gemisch 40 Minuten nachgerührt. Zu dieser dunkelroten Ylid-Lösung wird eine Lösung von 5.03 g des im Beispiel 7 hergestellten Lactols in 305 ml abs. Tetrahydrofuran zugetropft und nach Beendigung der Zugabe wird das Gemisch 2 Stunden unter Argon bei 45 °C gerührt. Die Lösung wird anschließend in 312 %iger Natriumchlorid-Lösung gegossen und durch Zugabe von 10 % Zitronensäure-Lösung wird das Gemisch auf pH 5-4.5 angesäuert. Die wässrige Phase wird 5 mal mit je 300 ml Ether ausgezogen und die zusammengefassten Ether-Phasen werden 5 mal mit je 40 ml 2 % Natriumhydroxid-Lösung extrahiert. Die Lauge-Extrakte werden zusammengefasst, mit 10 %iger Zitronensäure-Lösung auf pH 4.5 angesäuert und mit Ether mehrmals ausgezogen. Die Etherphasen werden über Magnesiumsulfat getrocknet und eingeengt. Um den Methylester zu erhalten wird das Rohprodukt in 200 ml Ether gelöst und mit einer etherischen Lösung von Diazomethan behandelt. Überschüssiges Diazomethan wird durch tropfenweise Zugabe von Eisessig vernichtet und die Lösung wird im Vakuum eingeengt. Das Rohprodukt wird über 300 g Kieselgel in einem Hexan/15 % Essigester-Hexan-Gradienten chromatographiert. Man erhält 5,01 g der Titelverbindung als Öl.

IR : 3 450, 2 930, 1 730, 1 580, 1 460, 1 430, 1 340, 1 070 (breit), 1 020, 835, 775, 740, 695 $cm^{-1}$.

Beispiel 9

(5RS)-5-Jod-5-[(2RS,3aS,4S,5R,6aS)-3a-phenylselenyl-4-tert.-butyldimethylsilyloxymethyl-5-(tetrahydropyran-2-yloxy)-perhydrocyclopenta [b] furan-2-yl]-pentansäuremethylester

Eine Lösung von 4,98 g des im Beispiel 8 hergestellten Olefins in 94.6 ml Ether wird mit einer Lösung von 10 g Natriumbicarbonat in 169 ml Wasser versetzt und das Gemisch auf 3 °C gekühlt. Innerhalb von

20 Minuten wird eine Lösung von 4,25 g Jod in 145 ml Ether zu diesem zweiphasen Gemisch zugetropft und die Lösung 2,5 Stunden bei 2-3 °C gerührt. Anschliessend werden die Phasen getrennt und die wässrige Lösung mit Ether extrahiert. Die organischen Phasen werden zusammengefasst, mit 5 % Natriumthiosulfat-Lösung und halbgesättigter Natriumchlorid-Lösung gewaschen. Die Ether-Lösung wird über Magnesiumsulfat getrocknet und im Vakuum eingeengt. Das Rohprodukt wird über 300 g Kieselgel in einem Hexan/10 % Essigester-Hexan-Gradienten chromatographiert. Man erhält 5.65 g der Titelverbindung als Öl.

IR : 2 930, 1 730, 1 580, 1 430, 1 350, 1 240, 1 120, 1 020, 975, 835, 775, 740 695 $cm^{-1}$.

## Beispiel 10

(5RS)-5-Jod-5-[(2RS,4S,5R,6aS)-4-tert.-butyldimethylsilyloxymethyl-5-(tetrahydropyran-2-yloxy)-4,5,6,6a-tetrahydro-2H-cyclopenta [b] furan-2-yl]-pentansäure-methylester

Eine Lösung von 4.52 g des im Beispiel 9 beschriebenen Jodethers in 150 ml Tetrahydrofuran wird mit 60 ml 15 % Wasserstoffperoxid-Lösung versetzt und das Gemisch 1 Stunde bei Raumtemperatur unter Argon gerührt. Die Lösung wird anschliessend in Eiswasser gerührt, die wässrige Lösung nacheinander mit Ether (3x) und Essigester (1x) extrahiert. Die organischen Phasen werden zusammengefasst und mit halbgesättigter Natriumchlorid-Lösung gewaschen. Die organische Lösung wird über Magnesiumsulfat getrocknet und im Vakuum eingeengt. Der Rückstand wird über 250 g Kieselgel in einem Hexan/12 % Essigester-Hexan-Gradienten chromatographiert. Man erhält 3.33 g der Titelverbindung als Öl.

IR : 2 900, 1 730, 1 440, 1 370, 1 240, 1 160, 1 100, 1 030, 965, 835, 775 $cm^{-1}$.

## Beispiel 11

5-[(2EZ)-(4S, 5R, 6aS)-4-tert.-Butyldimethylsilyloxymethyl-5-(tetrahydropyran-2-yloxy)-4,5,6a-tetrahydro-2H-cyclopenta [b] furan-2-yliden]-pentansäure-methylester

Eine Lösung von 2.33 g des im Beispiel 10 beschriebenen Jodethers in 47 ml Benzol wird mit 2.23 ml Diazabicycloundecene versetzt und das Gemisch 2 Stunden unter Argon bei 50 °C gerührt. Die Reaktionslösung wird mit Essigester verdünnt und mit Wasser 3mal gewaschen. Die organische Phase wird über Magnesiumsulfat getrocknet und eingeengt. Das Rohprodukt (1.86 g) wird ohne weitere Aufreinigung zur nächsten Stufe eingesetzt.

IR : 2 940, 1 730, 1 680, 1 440, 1 370, 1 240, 1 160, 1 100, 1 030, 965, 835, 775 $cm^{-1}$.

## Beispiel 12

5-[(4S,5R)4-tert.-Butyldimethylsilyloxymethyl-5-(tetrahydropyran-2-yloxy)-5,6-dihydro-4H-cyclopenta-[b]furan-2-yl]-pentansäure-methylester

Eine Lösung von 1.86 g des im Beispiel 11 hergestellten Dienolethers in 180 ml Benzol wird unter Argon mit 5.52 g Magnesiumsulfat (getr.) versetzt und das Gemisch 2 Stunden bei Rückflusstemperatur gehalten. Man lässt die Lösung abkühlen, das Magnesiumsulfat wird abgesaugt und mit Essigester gewaschen. Das Filtrat wird im Vakuum eingeengt und auf 150 g Kieselgel in einem Hexan/10 % Essigester-Hexan-Gradienten chromatographiert. Man erhält 1.20 g der Titelverbindung als Öl.

IR : 2 930, 1 730, 1 620 (schwach), 1 550, 1 460, 1 430, 1 340, 1 240, 1 190, 1 110, 1 070, 1 030, 980, 935, 775 $cm^{-1}$.

## Beispiel 13

5-[(4S,5R)-4-Hydroxymethyl-5-(tetrahydropyran-2-yloxy)-5,6-dihydro-4H-cyclopenta[b]furan-2-yl]-pentansäuremethylester

Eine Lösung von 900 mg des im Beispiel 12 hergestellten Furan-Derivats in 120 ml abs. Tetrahydrofuran wird mit 960 mg Tetrabutylammoniumfluorid gelöst in 10 ml Tetrahydrofuran versetzt und das Gemisch 1 Stunde bei Raumtemperatur unter Argon gerührt. Anschliessend wird die Reaktionslösung in Eiswasser gefällt und die wässrige Phase mehrmals mit Ether ausgezogen. Die organischen Phasen werden zusammengefasst, mit Wasser und anschliessend mit halbgesättigter Natriumchlorid-Lösung gewaschen, über Magnesiumsulfat getrocknet und eingeengt. Das Rohprodukt wird über 60 g Kieselgel in einem Hexan/20 % Aceton-Hexan-Gradienten chromatographiert. Man erhält 648 mg der Titelverbindung als Öl.

IR : 3 900, 2 920, 1 720, 1 620, 1 540, 1 430, 1 340, 1 190, 1 130: 1 060, 1 030, 870, 805 $cm^{-1}$.

Beispiel 14

5-[(4R,5R)-4-Formyl-5-(tetrahydropyran-2-yloxy)-5,6-dihydro-4H-cyclopenta[b]furan-2-yl]-pentansäure-methylester .

Zu einer Lösung von 590 mg des im Beispiel 13 hergestellten Alkohols in 75 ml Methylenchlorid werden 271 mg Natriumacetat und 534 mg. Pyridiniumchlorochromat zugegeben und das Gemisch wird 50 Minuten unter Argon gerührt. Das Gemisch wird mit einer Lösung von 0.25 ml Isopropanol und 0.5 ml Pyridin in 50 ml Ether versetzt und 10 Minuten weiter gerührt. Anschliessend werden 7.5 g Magnesiumsulfat eingetragen, die Reaktionslösung filtriert und der Rückstand mit Ether gewaschen. Die organische Lösung wird direkt auf eine 30 g Kieselgel-Säule aufgetragen und mit einem Hexan/8 % Aceton-Hexan-Gradienten eluiert. Man erhält 325 mg der Titelverbindung als Öl.

IR : 2 930, 2 700 (schwach), 1 730, 1 650 (schwach), 1 550, 1 430, 1 340, 1 250, 1 190, 1 130, 1 070, 1 030, 960 (schwach), 865, 815 $cm^{-1}$.

Beispiel 15

5-{(4R,5R)-4-[(E)-3-Oxo-1-octenyl]-5-(tetrahydropyran-2-yloxy)-5,6-dihydro-4H-cyclopenta[b]furan-2-yl}-pentan-säure-methylester

Eine Suspension von 53 mg Natriumhydrid (50 % in Öl) in 10 ml abs. Dimethoxyethan wird mit einer Lösung von 244 mg Dimethyl-(2-oxoheptyl)-phosphonat in 3.0 ml abs. Dimethoxyethan tropfenweise versetzt und das Gemisch 0.5 Stunden bei Raumtemperatur unter Argon gerührt. Die Lösung wird auf − 30 °C gekühlt, wobei das Natriumsalz des Phosphonats auskristallisiert. Zu dieser Suspension wird eine Lösung von 310 mg des in Beispiel 14 beschriebenen Aldehyds in 3,0 ml abs. Dimethoxyethan zugetropft und das Gemisch 90 Minuten bei − 30 °C gerührt. Anschliessend wird das Gemisch mit einer Lösung von 0.5 ml Eisessig in 3.0 ml Dimethoxyethan versetzt und mit Ether verdünnt. Diese Lösung wird nacheinander mit verdünnter Natriumbicarbonat-Lösung, Wasser und Sole gewaschen, über Magnesiumsulfat getrocknet und eingeengt. Das Rohprodukt (450 mg) wird mittels präparativer Schichtchromatographie auf 5 Platten (20 × 40 cm, Schichtdicke = 0.5 mm) in einem Chloroform-Ether (8 : 2)-System aufgereinigt. Man erhält 229 mg der Titelverbindung.

IR : 2 940, 1 740, 1 670, 1 630, 1 550, 1 430, 1 340, 1 200, 1 130, 1 070, 1 030, 980 $cm^{-1}$.

Beispiel 16

5-{(4R,5R)-4-[(3S)-(E)-3-Hydroxy-1-octenyl]-5-(tetrahydropyran-2-yloxy)-5,6-dihydro-4H-cyclopenta[b]furan-2-yl}-pentansäure-methylester und 5-{(4R, 5R)-4-[(3R)-(E)-3-Hydroxy-1-octenyl]-5-(tetrahydropyran-2-yloxy)-5,6-dihydro-4H-cyclopenta[b]furan-2-yl}-pentansäure-methylester

Eine Lösung von 198 mg des im Beispiel 15 beschriebenen Ketons in 5.3 ml abs. Methanol wird mit 100 mg Natriumborhydrid bei − 40 °C versetzt und das Gemisch 0.5 Stunden unter Argon gerührt. Anschliessend wird zur Reaktionslösung 0.14 ml Eisessig vorsichtig zugetropft und das Gemisch in Eiswasser gefällt. Die wässrige Phase wird mit Ether extrahiert, der organische Extrakt mit verdünnter Natriumbicarbonat-Lösung und anschliessend mit halbgesättigter Natriumchlorid-Lösung gewaschen. Die Ether-Lösung wird über Magnesiumsulfat getrocknet und im Vakuum eingeengt. Man erhält ein Rohprodukt (205 mg), das aus einem Gemisch der 15-Alkohole besteht. Zur Trennung der 15-Epimeren wird das Gemisch mittels präparativer Schichtchromatographie auf 3 Platten (20 × 40 cm, Schichtdicke 0,5 mm) in einem Chloroform-Ether (9 : 1)-System aufgereinigt. Man erhält 81,6 mg des 15α-Alkohols (Prostaglandin-Nummerierung).

IR : 3 950, 2 930, 1 730, 1 630 (schwach), 1 540, 1 430, 1 340, 1 210, 1 130, 1 070, 1 030, 965 $cm^{-1}$ und 87.4 mg des entsprechenden 15β-Alkohols.

IR : 3 950, 2 930, 1 730, 1 630 (schwach), 1 540, 1 430, 1 340, 1 210, 1 130, 1 070, 1 030, 965 $cm^{-1}$.

Beispiel 17

5-{(4R,5R)-4-[(3S)-(E)-3-Hydroxy-1-octenyl]-5-hydroxy-5,6-dihydro-4H-cyclopenta[b]furan-2-yl}-pentansäuremethylester (Furaprostacyclin-methylester) und 5-{(4R, 5R)-4-[(3R)-(E)-3-Hydroxy-1-octenyl]-5-hydroxy-5,6-dihydro-4H-cyclopenta[b]furan-2-yl}-pentansäure-methylester

Eine Lösung von 78.1 mg des im Beispiel 17 beschriebenen 15α-Alkohols wird in 7.7 ml einer Tetrahydrofuran/Eisessig/Wasser-Mischung (35 : 65 : 10) gelöst und das Gemisch 6 Stunden unter Argon gerührt. Anschliessend wird das Lösungsmittel im Vakuum bei Raumtemperatur abgezogen, der Rückstand in Toluol gelöst und die Lösung abdestilliert. Das Rohprodukt (60 mg) wird mittels Schichtchromatographie auf vier analytischen Dünnschichtplatten (Fa. Merck, 20 × 20 cm, Schichtdicke

0,25 mm) in einem Chloroform-Ether (9 : 1)-System aufgereinigt. Man erhält 54.4 mg des 15α-Alkohols (Prostaglandin-Nummerierung).

IR : 3 700, 2 920, 1 730, 1 620 (schwach), 1 550, 1 430, 1 330, 1 190, 1 070, 970, 935, 795 $cm^{-1}$.

Das 15β-Alkohol-Epimere (64 mg) wird analog behandelt. Man erhält 51 mg des 11α,15β-Diols.

IR : 3 700, 2 920, 1 730, 1 620 (schwach), 1 550, 1 430, 1 330, 1 190, 1 070, 970, 935, 795 $cm^{-1}$.

## Beispiel 18

5-{(4R,5R)-4-[(3S)-(E)-3-Hydroxy-1-octenyl]-5-hydroxy-5,6-dihydro-4H-cyclopenta[b]furan-2-yl}-pentansäure(Furaprostacyclin) und 5-{(4R,5R)-4-[(3R)-(E)-3-Hydroxy-1-octenyl]-5-hydroxy-5,6-dihydro-4H-cyclopenta[b]furan-2yl}-pentansäure

Eine Lösung von 30.8 mg des im Beispiel 17 hergestellten 11α,15α-Diols in 1.6 ml Methanol wird mit 0.16 ml 10 % Kaliumhydroxid-Lösung versetzt und das Gemisch 9 Stunden bei Raumtemperatur unter Argon gerührt. Anschliessend wird das Lösungsmittel bei 22 °C im Vakuum weitgehend eingeengt und der Rückstand in 2.5 ml Wasser gelöst. Die wäßrige Phase wird mit Ether extrahiert, die organische Phase abgetrennt und verworfen. Die wäßrige Phase wird mit 10 %iger Zitronensäure-Lösung angesäuert (pH 5-4.5) und mit Ether extrahiert. Die Ether-Lösung wird über Magnesiumsulfat getrocknet und eingeengt. Man erhält 26 mg der Titelverbindung (15α-Alkohol-Epimere).

IR : 3 900 (breit), 2 950, 1 720, 1 620 (schwach), 1 550, 1 430, 1 330, 970 $cm^{-1}$.

Der im Beispiel 17 beschriebene 11α,15β-Diol-methylester wird analog in methanolischer Kalilauge verseift. Ausgehend von 36 mg Ester erhält man 28.5 mg der Titelverbindung (15β-Alkohol-Epimere).

IR : 3 900 (breit), 2 950, 1 720, 1 620 (schwach), 1 550, 1 430, 1 330, 970 $cm^{-1}$.

## Beispiel 19

5-{(4R,5R)-4-[(E)-(4RS)-3-Oxo-4-methyl-1-octenyl]-5-(tetrahydropyran-2-yloxy)-5,6-dihydro-4H-cyclopenta[b]furan-2-yl}-pentansäure-methylester

Eine suspension von 51 mg Natriumhydrid (50 % Suspension in Öl) in 8.5 ml abs. Dimethoxyäthan wird mit einer Lösung von 246 ml Dimethyl-(2-oxo-3-methylheptyl)-phosphonat (DE-OS 22 21 301) inl 2.0 ml abs. Dimethoxyethan tropfenweise versetzt und das Gemisch 0.5 Stunden bei Raumtemperatur unter Argon gerührt. Die Lösung wird auf − 40 °C abgekühlt, mit einer Lösung von 332 mg des im Beispiel 14 beschriebenen Aldehyds in 3.0 mg abs. Dimethoxymethan versetzt und das Gemisch 2 Stunden bei − 30 °C gerührt. Zu der Reaktionslösung werden 0.55 ml Eisessig in 3.0 ml Dimethoxyethan zugetropft und das Gemisch wird anschliessend mit ca. 20 ml Äther verdünnt. Diese Lösung wird nacheinander mit verdünnter Natriumbicarbonat-Lösung, Wasser und halb-gesättigter Natriumchlorid-Lösung gewaschen, über Magnesiumsulfat getrocknet und im Vakuum eingeengt. Das Rohprodukt wird über 20 g Kieselgel in einem Hexan/20 % Essigester-Hexan-Gradienten chromatographiert. Man erhält 271 mg der Titelverbindung als Öl.

IR : 2 940, 1 740, 1 670, 1 640, 1 550, 1 430, 1 340, 1 200, 1 130, 1 070, 1 030, 970 $cm^{-1}$.

## Beispiel 20

5-{(4R,5R)-4-[(3S,4RS)-(E)-3-Hydroxy-4-methyl-1-octenyl]-5-hydroxy-5,6-dihydro-4H-cyclopenta[b]furan-2-yl}-pentansäure (16-methylfuraprostacyclin)

Analog den Beispielen 16-18 erhält man ausgehend von 260 mg des im Beispiel 19 erhaltenen α,β-ungesättigten Ketons 66 mg der Titelverbindung.

IR : 3 900 (breit), 2 950, 1 720, 1 620, 1 540, 1 430, 1 330, 970 $cm^{-1}$.

## Beispiel 21

5-{(4R,5R)-4-[(5RS)-(E)-3-Oxo-4-fluor-1-octenyl]-5-(tetrahydropyran-2-yloxy)-5,6-dihydro-4H-cyclopenta[b]furan-2-yl}-pentansäure-methylester

Zu einer Suspension von 55 mg Natriumhydrid (50 % Suspension in Öl) in 12.0 ml abs. Dimethoxyethan wird eine Lösung von 288 mg Dimethyl-(2-oxo-3-fluorheptyl)-phosphonat (DE-OS 23 20 368) in 4.5 ml abs. Dimethoxyethan innerhalb von 5 Minuten bei Raumtemperatur unter Argon gerührt. Die Lösung wird auf − 30 °C abgekühlt, mit einer Lösung von 420 mg des im Beispiel 14 beschriebenen Aldehyds in 4.5 ml abs. Dimethoxyethan versetzt und das Gemisch 2 Stunden bei − 40 °C gerührt. Zur Reaktionslösung werden anschliessend 0.60 ml Essigsäure im 4.0 ml Dimethoxyethan zugetropft und das Gemisch wird mit Äther verdünnt. Die organische Lösung wird nacheinander mit verdünnter Natriumbicarbonat-Lösung, Wasser und halbgesättigter Natriumchlorid-Lösung gewaschen, über Magnesiumsulfat getrocknet und im Vakuum eingeengt. Das Rohprodukt wird über 20 g Kieselgel in einem Hexan/25 % Essigester-Hexan-Gradienten chromatographiert. Man erhält 345 mg der Titelverbindung.
IR(Chloroform) : 2 950, 1 720, 1 700 (Schulter), 1 625, 1 550, 1 430, 1 330, 970 $cm^{-1}$.

Beispiel 22

5-{(4R,5R)-4-[(3R,4RS)-(E)-3-Hydroxy-4-fluor-1-octenyl]-5-hydroxy-5,6-dihydro-4H-cyclopenta[b]furan-2-yl}-pentansäure

Analog den Beispielen 16-18 erhält man ausgehend von 340 mg des im Beispiel 21 erhaltenen α,β-ungesättigten Ketons 74 mg der Titelverbindung.
IR : 3 900 (breit), 2 950, 1 720, 1 620, 1 540, 1 430, 1 320, 970 $cm^{-1}$.

Beispiel 23

5-{(4R,5R)-4-[(E)-3-Oxo-4-phenoxy-1-butenyl]-5-(tetrahydropyran-2-yloxy)-5,6-dihydro-4H-cyclopenta[b]furan-2-yl}-pentansäure-methylester

Zu einer auf − 30 °C abgekühlten Lösung von 290 mg des im Beispiel 14 beschriebenen Aldehyds in 18.5 ml abs. Tetrahydrofuran werden 405 mg des Lithiumsalzes von Dimethyl-(2-oxo-3-phenoxypropyl)-phosphonat (DE-OS 26 55 004) eingetragen und die Lösung wird 1,5 Stunden bei dieser Temperatur unter Argon gerührt. Anschliessend lässt man die Lösung auf 5 °C erwärmen und fügt 1.1 ml Essigsäure hinzu. Die Lösung wird mit Äther verdünnt und nacheinander mit verdünnter Natriumbicarbonat-Lösung, Wasser und halb-gesättigter Natriumchlorid-Lösung gewaschen. Die organische Phase wird über Magnesiumsulfat getrocknet und im Vakuum eingeengt. Nach Aufreinigung des Rohproduktes mittels Chromatographie über Kieselgel (20 g) in einem Hexan/20 % Essigester-Hexan-Gradienten erhält man 303 mg der Titelverbindung.
IR : 2 940, 1 730, 1 670, 1 630, 1 600, 1 540, 1 430, 1 330, 980, 760 $cm^{-1}$.

Beispiel 24

5-{(4R,5R)-4-[(3R)-(E)-3-Hydroxy-4-phenoxy-1-butenyl]-5-hydroxy-5,6-dihydro-4H-cyclopenta[b]furan-2-yl}-pentansäure

Analog den Beispielen 16-18 erhält man ausgehend von 300 mg des im Beispiel 23 erhaltenen α,β-ungesättigten Ketons 62 mg der Titelverbindung.
IR : 3 900 (breit), 2 950, 1 730, 1 670, 1 640, 1 600, 1 540, 1 430, 1 330, 980, 760 $cm^{-1}$.

Beispiel 25

5-{(4R,5R)-4-[(4RS)-(1E,6Z)-3-Oxo-4-methyl-7-chlor-1,6-octadienyl]-5-(tetrahydropyran-2-yloxy)-5,6-dihydro-4H-cyclopenta[b]furan-2-yl}-pentansäure-methylester

Zu einer Suspension von 57 mg Natriumhydrid (50 % in Öl) in 12.0 ml abs. Dimethoxyethan werden 317 mg Dimethyl-[(5Z)-2-oxo-3-methyl-6-chlor-5-heptenyl]-phosphonat in 4.0 ml abs. Dimethoxymethan innerhalb von 10 Minuten zugetropft und das Gemisch insgesamt 0.5 Stunden bei Raumtemperatur unter Argon gerührt. Die Lösung wird auf − 40 °C abgekühlt und mit einer Lösung von 360 mg des im Beispiel 14 beschriebenen Aldehyds versetzt. Nach 2 stündigem Rühren bei − 30 °C wird das Gemisch mit Essigsäure neutralisiert und mit Äther verdünnt. Die organische Lösung wird nacheinander mit verdünnter Natriumbicarbonat-Lösung, Wasser und halb-gesättigter Natriumbicarbonat-Lösung, Wasser und halb-gesättigter Natriumchlorid-Lösung gewaschen, über Magnesiumsulfat getrocknet und im Vakuum eingeengt. Der Rückstand wird über 25 g Kieselgel in einem Hexan/15 % Aceton-Hexan-Gradienten chromatographiert. Man erhält 285 mg der Titelverbindung.
IR : 2 930, 1 740, 1 660, 1 630, 1 540, 1 420, 1 340, 1 200, 1 130, 1 070, 1 030, 970 $cm^{-1}$.
Das im obigen Beispiel verwendete Dimethyl-[(5Z)-2-oxo-3-methyl-6-chlor-5-heptenyl]-phosphonat wird wie folgt hergestellt :

a) 2-[2Z)-3-Chlor-2-butynyl]-2-methyl-malonsäure-diäthylester

11.5 g in kleine Stücke geschnittenes Natrium werden in einem mit Rührer, Rückflußkühler und Tropftrichter versehenen Dreihalskolben gegeben. Hierzu werden 250 ml abs. Äthanol so schnell zugetropft, daß die Lösung lebhaft siedet. Anschließend werden 87 g destillierter Methylmalonsäurediäthylester zur heißen Alkoholat-Lösung getropft. Nach Abkühlenlassen auf ca. 75 °C wird die Reaktionslösung unter auftretender Gelbfärbung mit 66 g 1,3-Dichlorbuten-(2) tropfenweise versetzt. Nach 2 Stunden Rühren unter Erhitzen wird die einen pH-Wert von 5-6 aufweisende und jetzt fast vollständig entfärbte Suspension vom ausgefallenen Natriumchlorid durch Filtration befreit. Das Filtrat wird

eingeengt und mit dem durch Waschen des Niederschlags anfallenden Methylenchlorid vereinigt. Die organische Lösung wird dann mit gesättigter Natriumchlorid-Lösung geschüttelt, über Magnesiumsulfat getrocknet, am Rotations-verdampfer eingeengt und im Vakuum fraktioniert. Man erhält 105 g des gewünschten Diesters. $K_{p2,5}$ = 110 °C.
IR (Film) : 1 738, 1 666, 1 160, 1 050/cm.

b) 2-[(2Z)-3-Chlor-2-butenyl]-2-methyl-malonsäure

46 g des in der vorigen Reaktionsstufe erhaltenen Diesters werden zusammen mit 33 g Kaliumhydroxid in 85 ml Äthanol und 45 ml Wasser 3.5 Stunden unter Rückfluss erhitzt. Nach Abziehen des lösungsmittels im Vakuum wird der Rückstand in 45 ml Wasser aufgenommen und unter Eiskühlung tropfenweise mit konz. Salzsäure bis zum pH-Wert 1 angesäuert. Anschliessend wird die wässrige Phase fünfmal mit je 200 ml Äther ausgeschüttelt. Die vereinigten Äther-Extrakte werden mit gesättigter Natriumchlorid-Lösung gewaschen, über Magnesiumsulfat getrocknet und im Vakuum zur Trockne eingeengt. Der Rückstand wird aus Benzol/Cyclohexan umkristallisiert. Man erhält 33.5 g der Disäure.
Fp. : 99-101 °C.
Ir (KBr) : 2 700, 2 650, 2 580, 1 700, 1 663, 1 238/cm.

c) (4Z)-5-Chlor-2-methyl-4-hexensäure

33.5 g der in der vorigen Reaktionsstufe erhaltenen Dicarbonsäure werden 4 Stunden auf 160 °C unter $CO_2$-Entwicklung erhitzt. Das Produkt wird anschliessend im Vakuum destilliert. Man erhält 24.3 g der Monocarbonsäure. $Kp_{13}$ = 133-135 °C.
IR (Film) : 2 660, 2 570, 1 710, 1 668, 1 243/cm.

d) (4Z)-5-Chlor-2-methyl-4-hexensäure-methylester

Zu einer Lösung von 24.3 g der nach obiger Vorschrift erhaltenen Carbonsäure in 450 ml Acetonitril werden nacheinander 153 ml N-Äthyldiisopropylamin und 307 ml Jodmethan getropft. Nach 4 stündigem Rühren bei Raumtemperatur wird die Reaktionslösung mit eiskalter gesättigter Natriumchlorid-Lösung versetzt und mit Essigester extrahiert. Die vereinigten organischen Phasen werden nacheinander mit Natriumbisulfat, Natriumhydrogencarbonat und gesättigter Natriumchlorid-Lösung gewaschen, über Natriumsulfat getrocknet und am Rotationsverdampfer eingeengt. Der Rückstand wird im Vakuum destilliert. Es werden 21.9 g des gewünschten Esters isoliert. $Kp_{13}$ = 81-83 °C.
IR (Film) : 1 738, 1 665, 1 195, 1 172/cm.

e) Dimethyl-[(5Z)-2-oxo-3-methyl-6-chlor-5-heptenyl]-phosphonat

Zu einer Lösung von 67.1 g des im Beispiel 25d beschriebenen Methanphosphonsäuredimethylesters in 840 ml abs. Tetrahydrofuran tropft man unter Argon bei − 60 °C 247.5 ml einer 2.02 m Butyllithium-Lösung in Hexan. Nach 15 Minuten wird eine Lösung von 44.16 g des nach obiger Vorschrift erhaltenen Esters in 100 ml abs. Tetrahydrofuran zugetropft. Man hält das Reaktionsgemisch 3.5 Stunden bei − 65 °C, über Nacht bei − 32 °C und ließ es schließlich sich auf Raumtemperatur erwärmen. Danach wird es mit 28.6 ml Eisessig versetzt und im Vakuum zur Trockne eingeengt. Der Rückstand wird in einem Zweiphasensystem aus 175 ml Wasser und 825 ml Äther verteilt, die organische Phase über Magnesiumsulfat getrocknet und am Rotationsverdampfer eingeengt. Der Eindampfrückstand wird durch Destillation bei 40 °C/0.1 mm von flüchtigen Nebenprodukten und unumgesetztem Edukt befreit und anschließend durch Säulenchromatographie an Kieselgel mit Hexan/50-100 % Essigester als Fließmittel gereinigt. Neben 13.8 g Edukt erhält man 36 g Phosphonat.
IR (Film) : 1 712, 1 666, 1 260, 1 032/cm.

Beispiel 26

5-{(4R,5R)-4-[(3S,4RS) — (1E,6Z)-3-Hydroxy-4-methyl-7-chlor-1,6-octadienyl]-5-hydroxy-5,6-dihydro-4H-cyclopenta[b]furan-2-yl}-pentansäure

Analog den Beispielen 16-18 werden 70 mg der Titelverbindung, ausgehend von 280 mg des im Beispiel 25 erhaltenen α,β-ungesättigten Ketons erhalten.
IR : 3 900 (breit), 2 950, 1 720, 1 620 (schwach), 1 550, 1 430, 970 $cm^{-1}$.

Beispiel 27

5-{(4R,5R)-4-[(4RS)-(E)-3-Oxo-4,7-dimethyl-1,6-octadienyl]-5-(tetrahydropyran-2-yloxy)-5,6-dihydro-4H-cyclopenta[b]furan-2-yl}-pentansäure-methylester

Zu einer Suspension von 100 mg Natriumhydrid (50 % Suspension in Öl) in 22 ml abs. Dimethoxyethan werden 510 mg Dimethyl-(2-oxo-3,6-dimethyl-5-heptenyl)-phosphonat in 9 ml abs. Dimethoxyethan innerhalb von 10 Minuten zugetropft und das Gemisch wird 30 Minuten bei Raumtemperatur unter Argon gerührt. Die Lösung wird auf – 40 °C abgekühlt und mit einer Lösung von 600 mg des im Beispiel 14 beschriebenen Aldehyds in 9 ml abs. Dimethoxyethan versetzt. Das Gemisch wird 2 Stunden bei – 30 °C gerührt und anschließend mit 1.1 ml Essigsäure versetzt und mit Äther verdünnt. Nach Isolierung und Aufreinigung des Rohproduktes, wie im Beispiel 21 beschrieben, erhält man 473 mg der Titelverbindung.

IR : 2 930, 1 740, 1 650, 1 630, 1 540, 1 430, 1 330, 1 200, 1 130, 1 030, 970 $cm^{-}$.

Das im obigen Beispiel verwendete Dimethyl-(2-oxo-3,6-dimethyl-5-heptenyl)-phosphonat wird wie folgt hergestellt :

a) 2-Äthoxycarbonyl-2,5-dimethyl-4-hexensäure-ethylester

In einen mit Rückflusskühler, Tropftrichter und Rührer versehenen Dreihalskolben werden 36.1 g Natrium (in kleine Stücke geschnitten) gegeben. Hierzu werden 800 ml abs. Äthanol so schnell zugetropft, dass die Lösung in lebhaftem Sieden bleibt. Zur heissen Alkoholat-Lösung tropft man 269.6 g frisch destillierten Methylmalonsäurediäthylester, rührt 1/2 Stunde bei 60 °C und gibt dan — ebenfalls tropfenweise — 241.7 g Dimethylallylbromid hinzu. Nach einer Stunde Rühren unter Erhitzen filtriert man das ausgefallene Natriumbromid ab, wäscht den Niederschlag und engt das Filtrat ein. Der Rückstand wird in Äther aufgenommen, mit gesättigter Natriumchlorid-Lösung neutral gewaschen, über Magnesiumsulfat getrocknet und am Rotationsverdampfer eingeengt. Der Eindampfrückstand wird an der Ölpumpe fraktioniert. Man erhält 266 g der Titelverbindung vom $Kp_7$ = 97-112 °C.

IR (Film) : 1 735, 1 245, 1 025, 860/cm.

b) 2,5-Dimethyl-4-hexensäure-äthylester

85,3 g 2-Äthoxycarbonyl-2,5-dimethyl-4-hexensäureäthylester werden in 645 ml Dimethylsulfoxid gelöst und nacheinander mit 29.7 g Lithiumchlorid und 6.3 ml destilliertem Wasser versetzt. Danach wird das Reaktionsgemisch insgesamt 13 Stunden auf 200 °C erhitzt und anschließend — nach Abkühlenlassen — auf 1l Eiswasser gegossen. Die wäßrige Phase wird dreimal mit je 500 ml Methylenchlorid extrahiert. Die vereinigten organischen Extrakte werden dann zweimal mit Wasser gewaschen, über Magnesium-sulfat getrocknet, am Rotationsverdampfer eingeengt und im Vakuum destilliert. Man isoliert 53.1 g vom $Kp_{13}$ = 75-78 °C.

IR (Film) : 1 735, 1 160, 1 050/cm.

c) Dimethyl-(2-oxo-3,6-dimethyl-5-heptenyl)-phosphonat

Zu einer Lösung von 59 g Methanphosphonsäuredimethylester in 400 ml abs. Tetrahydrofuran tropft man unter Argon bei – 60 °C 474,7 ml einer 1.61 m Butyllithium-Lösung in Hexan. Nach 15 Minuten Nachrüben wird eine Lösung von 34.05 g des im Beispiel 27b beschriebenen 2,5-Dimethyl-4-hexensäureäthylesters in 100 ml abs. Tetrahydrofuran zugetropft. Man läßt das Reaktionsgemisch innerhalb von 4 Stunden sich auf Raumtemperatur erwärmen und rührt es anschließend noch 3 Stunden. Danach wird es mit 26.5 ml Eisessig versetzt und im Vakuum eingeengt. Der Rückstand wird mit Äther/Wasser aufgenommen, die wässrige Phase mit festem Natriumchlorid versetzt und ausgeäthert. Die vereinigten organischen Phasen werden über Magnesiumsulfat getrocknet und am Rotationsverdampfer eingeengt. Der Verdampfungsrückstand wird durch Säulenchromatographie an Kieselgel mit Hexan/50-100 % Essigester als Fließmittel gereinigt. Man erhält 32 g der Tiltelverbindung.

IR : (Film) : 1 710, 1 260, 1·030/cm.

Beispiel 28

5-{(4R,5R)-4-[(3S,4RS)-(E)-3-Hydroxy-4,7-dimethyl-1,6-octadienyl]-5-hydroxy-5,6-dihydro-4H-cyclopenta[b]furan-2-yl}-pentansäure

Analog den Beispielen 16-18 werden 111 mg der Titelverbindung ausgehend von 465 mg des im Beispiel 27 hergestellten α,β-ungesättigten Ketons erhalten.

IR : 3 900 (breit), 2 950, 1 720, 1 630, 1 550, 1 430, 1 330, 970 $cm^{-1}$.

Beispiel 29

5-{(3R,5R)-4-[(3S)-(1E,5Z)-3-Hydroxy-1,5-octadienyl]-5-(tetrahydropyran-2-yloxy)-5,6-dihydro-4H-cyclopenta[b]furan-2-yl}-pentansäure-methylester

Eine Lösung von 904 mg (2S)-(4Z)-2-Hydroxy-4-heptenyltriphenyl-phosphoniumbromid [E.J. Corey et al., J. Am. Chem. Soc., 93 (6), 1490 (1971)] wird unter Argon auf – 70 °C abgekühlt und tropfenweise

innerhalb von 10 Minuten mit 3.2 ml Butyllithium-Lösung (1.2 M in Hexan) versetzt. Die Lösung wird auf − 30 °C erwärmt und 0.5 Stunden bei dieser Temperatur gerührt. Zu diesem Gemisch werden 480 mg des im Beispiel 14 beschriebenen Aldehyds in 5 ml abs. Tetrahydrofuran bei − 40 °C zugegeben und die Lösung 1.5 Stunden weiter gerührt. Während dieser Zeit läßt man die Reaktionstemperatur langsam auf − 25 °C steigen. Die Lösung wird anschließend mit 1 ml Essigsäure versetzt, mit Äther verdünnt und die organische Phase nacheinander mit verdünnter Natriumbicarbonat-Lösung, wasser und halb-gesättigter Natriumchlorid-Lösung gewaschen. Die Äther-Lösung wird über Magnesiumsulfat getrocknet und im Vakuum eingeengt. Der Rückstand wird über 20 g Kieselgel in einem Hexan/20 % Aceton-Hexan-Gradienten chromatographiert. Man erhält 177 mg der Titelverbindung.

IR : 3 940, 2 930, 1 730, 1 640, 1 550, 1 430, 1 340, 1 210, 1 130, 1 070, 1 030, 970 $cm^{-1}$.

## Beispiel 30

5-{(4R,5R)-4-[(3S)-(1E,5Z)-3-Hydroxy-1,5-octadienyl]-5-hydroxy-5,6-dihydro-4H-cyclopenta[b]furan-2-yl}-pentansäure

Analog den Beispielen 17-18 erhält man ausgehend von 170 mg des nach Beispiel 29 hergestellten Alkohols 81 mg der Titelverbindung.

IR : 3 900 (breit), 2 950, 1 720, 1 630, 1 550, 1 430, 1 340, 970 $cm^{-1}$.

## Beispiel 31

5-{(4R,5R)-4-[(4RS)-(E)-3-Oxo-4-methyl-1-octen-6-inyl]-5-tetrahydropyran-2-yloxy)-5,6-dihydro-4H-cyclopenta[b]furan-2-yl}-pentansäure-methylester

Eine Suspension von 68 mg Natriumhydrid (50 % Suspension in Öl) in 9.5 ml abs. Dimethoxyethan wird mit 327 mg dimethyl-(2-oxo-3-methyl-5-heptinyl)-phosphonat (DE-OS 27 29 960) in 4.5 ml abs. Dimethoxyethan tropfenweise innerhalb von 10 Minuten bei Raumtemperatur unter Argon versetzt und das Gemisch 0.5 Stunden gerührt. Anschließend wird die Lösung auf − 40 °C gekühlt und mit 430 mg des nach Beispiel 14 hergestellten Aldehyds in 5 ml abs. Dimethoxyethan versetzt und das Gemisch 2 Stunden bei − 30 °C gerührt. Anschließend wird zum Reaktionsgemisch 1.3 ml Essigsäure zugegeben, die Lösung mit Äther verdünnt und die organische Phase nacheinander mit verdünnter Natriumcarbonat-Lösung, Wasser und halb-gesättigter Natriumchlorid-Lösung gewaschen. Die Äther-Lösung wird über Magnesiumsulfat getrocknet und im Vakuum eingeengt. Der Rückstand wird auf Kieselgel in einem Hexan/15 % Essigester-Hexan-Gradienten chromatographiert. Man erhält 364 mg der Titelverbindung.

IR : 2 930, 1 730, 1 670, 1 630, 1 550, 1 430, 1 340, 1 120, 1 030, 980 $cm^{-1}$.

## Beispiel 32

5-{(4R,5R)-4-[(3S,4RS)-(E)-3-Hydroxy-4-methyl-1-octen-6-inyl]-5-(tetrahydropyran-2-yloxy)-5,6-dihydro-4H-cyclopenta[b]furan-2-yl}-pentansäure-methylester und

5-{(4R,5R)-4-[(3R,4RS)-(E)-3-Hydroxy-4-methyl-1-octen-6-inyl]-5-(tetrahydropyran-2-yloxy)-5,6-dihydro-4H-cyclopenta[b]furan-2-yl}-pentansäure-methylester

Analog Beispiel 16 erhält man durch Reduktion von 350 mg des im Beispiel 31 beschriebenen α,β-ungesättigten Ketons mit Natriumborhydrid 139 mg des 15α-Alkohols,

IR : 3 950, 2 940, 1 730, 1 630 (schwach), 1 540, 1 430, 1 340, 1 210, 1 060, 1 030, 970 $cm^{-1}$ und 147 mg des 15β-Alkohols.

IR : 3 950, 2 940, 1 730, 1 630 (schwach), 1 540, 1 430, 1 340, 1 210, 1 060, 1 030, 970 $cm^{-1}$.

## Beispiel 33

5-{(4R,5R)-4-[(3S,4RS)-(E)-3-Hydroxy-4-methyl-1-octen-6-inyl]-5-hydroxy-5,6-dihydro-4H-cyclopenta[b]furan-2-yl}-pentansäure und

5-{(4R,5R)-4-[(3R,4RS)-(E)-3-Hydroxy-4-methyl-1-octen-6-inyl]-5-hydroxy-5,6-dihydro-4H-cyclopenta[b]furan-2-yl}-pentansäure

Analog den Beispielen 17-18 erhält man ausgehend von 135 mg des im Beispiel 32 beschriebenen 11-Tetrahydropyranyläther-15α-ols 106 mg der Titelverbindung (11α,15α-Diol).

IR : 3 900 (breit), 2 950, 1 720, 1 620 (schwach), 1 540, 1 430, 1 330, 1 210, 970 $cm^{-1}$.

Unter analogen Bedingungen erhält man ausgehend von 147 mg des im Beispiel 32 beschriebenen 11-Tetrahydropyranyläther-15β-ols 95 mg der Titelverbindung (11α,15β-Diol).

Beispiel 34

5-{(4R,5R)-4-[(4RS)-(E)-3-Oxo-4-methyl-1-nonen-7-inyl]-5-(tetrahydropyran-2-yloxy)-5,6-dihydro-4H-cyclopenta[b]furan-2-yl}-pentansäure-methylester

Analog Beispiel 31 wird eine Lösung vom Natriumsalz des Dimethyl-(2-oxo-3-methyl-6-octinyl)-phosphonats durch Behandlung einer Suspension von 56 mg NaH (50 % Suspension in Öl) in abs. Dimethoxyethan (10 ml) mit 286 mg Dimethyl-(2-oxo-3-methyl-6-octinyl)-phosphonat (DE-OS 30 48 906) in 5 ml abs. Dimethoxyethan hergestellt. Diese Lösung wird mit 360 mg des nach Beispiel 14 hergestellten Aldehyds versetzt und man erhält nach Isolierung und Aufreinigung des Rohprodukts 184 mg der Titelverbindung.

IR : 2 940, 1 740, 1 670, 1 630, 1 550, 1 430, 1 340, 1 120, 1 030, 980 $cm^{-1}$.

Beispiel 35

5-{(4R,5R)-4-[(3S,4RS)-(E)-3-Hydroxy-4-methyl-1-nonen-7-inyl]-5-hydroxy-5,6-dihydro-4H-cyclopenta[b]furan-2-yl}-pentansäure

Analog den Beispielen 16-18 erhält man ausgehend von 180 mg des im Beispiel 34 beschriebenen $\alpha,\beta$-ungesättigten Ketons 44 mg der Titelverbindung.

IR : 3 900 (breit), 2 940, 1 725, 1 620 (schwach), 1 550, 1 430, 1 330, 970 $cm^{-1}$.

Beispiel 36

5-[(4R,5R)-4-[(3S)-(E)-3-Hydroxy-1-octenyl]-5-hydroxy-5,6-dihydro-4H-cyclopenta[b]furan-2-yl}-pentansäure-tris-(hydroxymethyl)-aminomethansalz

Eine Lösung von 35 mg der im Beispiel 18 beschriebenen $11\alpha,15\alpha$-Dihydroxysäure in 2.0 ml Acetonitril wird unter Argon auf 40 °C erwärmt und mit einer Lösung von 12 mg Tris-(hydroxymethyl)-aminomethan in 0.2 ml Wasser versetzt. Das Gemisch wird über Nacht bei Raumtemperatur gerührt und anschliessend wird die Lösung im Vakuum zur Trockne eingeengt. Man erhält 47 mg der Titelverbindung.

Beispiel 37

5-{(4R,5R)-4-[(3S,4RS)-(E)-3-Hydroxy-4-methyl-1-octenyl]-5-hydroxy-5,6-dihydro-4H-cyclopenta[b]furan-2-yl}-pentansäuremethylsulfonylamid

Eine Lösung von 37 mg der nach beispiel 20 hergestellten Säure in 0.3 ml Pyridin wird mit 0.1 ml Essigsäureanhydrid versetzt und das Gemisch 15 Stunden bei Raumtemperatur unter Argon gerührt. Die Lösung wird im Vakuum eingeengt und der Rückstand in 1.5 ml Acetonitril gelöst. Zu dieser Lösung werden 12 mg Triethylamin und eine Lösung von 16 mg Methylsulfonylisocyanat in 0.8 ml Acetonitril zugegeben.

Nach 4 stündigem Rühren bei Raumtemperatur wird die Lösung in Wasser (2 ml) gegossen und die wäßrige Phase mit 10 %iger Zitronensäure-Lösung neutralisiert. Diese Lösung wird mit Ether ausgezogen, die organische Phase mit halb-gesättigter Natriumchlorid-Lösung gewaschen, über Magnesiumsulfat getrocknet und im Vakuum eingeengt. Der Rückstand wird mittels präparativer Schichtchromatographie auf 3 Kieselgel-Fertigplatten (Fa. Merck, 20 × 20 cm, Schichtdicke 0.25 mm) in Ether als Laufmittel aufgereinigt. Man erhält 33 mg des Methylsulfonamids. Die Acetat-Schutzgruppen werden durch Umesterung in Methanol (1 ml)/Kaliumcarbonat (30 mg) entfernt. Nach 3 stündigem Rühren unter Argon wird die Methanol-Lösung neutralisiert und mit Methylenchlorid verdünnt. Die organische Phase wird mit halbgesättigter Natriumchlorid-Lösung gewaschen, über Magnesiumsulfat getrocknet und eingeengt. Der Rückstand wird mittels präparativer Schichtchromatographie auf 2 Fertigplatten (wie oben) in Chloroform-Ethanol 9:1 als Laufmittel aufgereinigt. Man erhält 20 mg der Titelverbindung als Öl.

IR : ($CHCl_3$) : 3400, 1720 (breit), 1550, 1430, 1340, 975 $cm^{-1}$.

Beispiel 38

5-{(4R,5R)-4-[(3S,4RS)-(E)-3)-Hydroxy-4-methyl-1-octen-6-inyl]-5-hydroxy-5,6-dihydro-4H-cyclopenta [b] furan-2-yl}-pentansäure-Kaliumsalz

Eine Lösung von 60 mg des im Beispiel 33 beschriebenen Prostacyclin-Derivats wird in 1,5 ml Methanol gelöst und mit 1,56 ml 0,107 M Kaliumhydrazid-Lösung unter Argon versetzt. Die Lösung wird 15 Minuten gerührt und anschliessend im Vakuum bei Raumtemperatur eingeengt. Man erhält 64 mg der Titelverbindung als weisses Pulver.

Schmp. 42-51 °C.

**Patentansprüche**

1. Prostacyclinderivate der allgemeinen Formel I

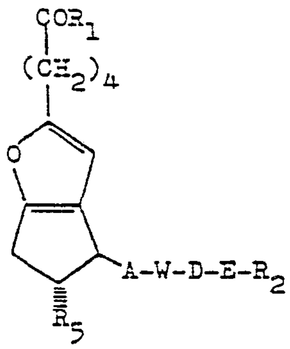

(I)

worin

$R_1$ den Rest $OR_3$, wobei $R_3$ Wasserstoff oder gegebenenfalls durch Halogen, Aryl, $C_1$-$C_4$-Alkoxy oder $C_1$-$C_4$-Dialkylamino substituiertes Alkyl mit 1-10 C-Atomen, Cycloalkyl, Aryl oder einen heterocyclischen Rest bedeuten kann, oder den Rest $NHR_4$ mit $R_4$ in der Bedeutung eines Wasserstoff, Alkanoyl- oder Alkansulfonylrestes mit je 1-10 C-Atomen darstellt,

A eine $-CH_2-CH_2-$ oder trans $-CH{=}CH$-Gruppe

W eine 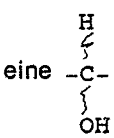 oder eine 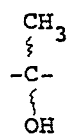 Gruppe,

in denen die OH-Gruppe jeweils mit einem Benzoyl oder Alkansäurerest mit 1-4 C-Atomen verestert oder mit einem Tetrahydropyranyl-, Tetrahydrofuranyl-, $C_1$-$C_4$-Alkoxyalkyl- oder Tri-($C_1$-$C_4$-alkyl)-silylrest veräthert sein kann, wobei die freie oder veresterte OH-Gruppe $\alpha$- oder $\beta$-ständig sein kann,

D und E gemeinsam eine direkte Bindung oder

D eine geradkettige oder verzweigte Alkylengruppe mit 1-5 C-Atomen,

E ein Sauerstoffatom oder eine direkte Bindung,

$R_2$ eine gerad- oder verzweigtkettige Alkylgruppe mit 1-6 C-Atomen, eine gerad- oder verzweigtkettige Alkenylgruppe mit 2-6 C-Atomen, die durch Phenyl, Halogen oder $C_1$-$C_4$-Alkyl substituiert sein kann und wenn D und E gemeinsam eine Direktbindung bedeuten, einen gegebenenfalls in 1-Stellung durch Halogen oder $C_1$-$C_4$-Alkyl substituierten Alkinylrest mit 2-6 C-Atomen, oder, wenn $R_1$ eine Hydroxygruppe, $R_5$ eine Hydroxygruppe, A eine trans $-CH{=}CH$-Gruppe,

W eine 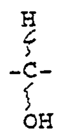 Gruppe

D eine $-CH_2$-Gruppe und E ein O-Atom bedeuten, Phenyl,

$R_5$ eine Hydroxygruppe, die mit einem Alkansäurerest mit 1-4 C-Atomen verestert oder mit einem Tetrahydropyranyl-, Tetrahydrofuranyl-, Alkoxyalkyl- oder Trialkylsilylrest veräthert sein kann und, falls $R_3$ Wasserstoff darstellt, deren Salze mit physiologisch verträglichen Basen bedeuten.

2. 5-{(4R,5R)-4-[(3S)-(E)-3-Hydroxy-1-octenyl]-5-(tetrahydropyran-2-yloxy)-5,6-dihydro-4H-cyclopenta [b] furan-2-yl}-pentansäure-methylester.

3. 5-{(4R,5R)-4-[(3R)-(E)-3-Hydroxy-1-octenyl]-5-(tetrahydropyran-2-yloxy)-5,6-dihydro-4H-cyclopenta [b] furan-2-yl}-pentansäure-methylester.

4. 5-{(4R,5R)-4-[(3S)-(E)-3-Hydroxy-1-octenyl}-5-hydroxy-5,6-dihydro-4H-cyclopenta [b] furan-2-yl}-pentansäure-methylester.

5. 5-{(4R,5R)-4-[(3R)-(E)-3-Hydroxy-1-octenyl]-5-hydroxy-5,6-dihydro-4H-cyclopenta [b] furan-2-yl}-pentansäure-methylester.

6. 5-{(4R,5R)-4-[(3S)-(E)-3-Hydroxy-1-octenyl]-5-hydroxy-5,6-dihydro-4H-cyclopenta [b] furan-2-yl}-pentansäure.

7. 5-{(4R,5R)-4-[(3R)-(E)-3-Hydroxy-1-octenyl]-5-hydroxy-5,6-dihydro-4H-cyclopenta [b] furan-2-yl}-pentansäure.

8. 5-{(4R,5R)-4-[(3S,4RS)-(E)-3-Hydroxy-4-methyl-1-octenyl]-5-hydroxy-5,6-dihydro-4H-cyclopenta [b]-furan-2-yl}-pentansäure.

9. 5-{(4R,5R)-4-[(3R,4RS)-(E)-3-Hydroxy-4-fluor-1-octenyl]-5-hydroxy-5,6-dihydro-4H-cyclopenta [b]-furan-2-yl}-pentansäure.

10. 5-{(4R,5R)-4-[(3R)-(E)-3-Hydroxy-4-phenoxy-1-butenyl]-5-hydroxy-5,6-dihydro-4H-cyclopenta [b]-furan-2-yl}-pentansäure.

11. 5-{(4R,5R)-4-[(3S,4RS)-(1E,6Z)-3-Hydroxy-4-methyl-7-chlor-1,6-octadienyl]-5-hydroxy-5,6-dihydro-4H-cyclopenta [b] furan-2-yl}-pentansäure.

12. 5-{(4R,5R)-4-[(3S,4RS)-(E)-3-Hydroxy-4,7-dimethyl-1,6-octadienyl]-5-hydroxy-5,6-dihydro-4H-cyclopenta [b] furan-2-yl}-pentansäure.

13. 5-{(4R,5R)-4-[(3S)-(1E,5Z)-3-Hydroxy-1,5-octadienyl]-5-(tetrahydropyran-2-yloxy)-5,6-dihydro-4H-cyclopenta [b] furan-2-yl}-pentansäure-methylester.

14. 5-{(4R,5R)-4-[(3S)-(1E,5Z)-3-Hydroxy-1,5-octadienyl]-5-hydroxy-5,6-dihydro-4H-cyclopenta [b]-furan-2-yl}-pentansäure.

15. 5-{(4R,5R)-4-[(3S,4RS)-(E)-3-Hydroxy-4-methyl-1-octen-6-inyl]-5-(tetrahydropyran-2-yloxy)-5,6-dihydro-4H-cyclopenta [b] furan-2-yl}-pentansäuremethylester.

16. 5-{(4R,5R)-4-[(3R,4RS)-(E)-3-Hydroxy-4-methyl-1-octen-6-inyl]-5-(tetrahydropyran-2-yloxy)-5,6-dihydro-4H-cyclopenta [b] furan-2-yl}-pentansäuremethylester.

17. 5-{(4R,5R)-4-[(3S,4RS)-(E)-3-Hydroxy-4-methyl-1-octen-6-inyl]-5-hydroxy-5,6-dihydro-4H-cyclopenta [b] furan-2-yl}-pentansäure.

18. 5-{(4R,5R)-4-[(3R,4RS)-(E)-3-Hydroxy-4-methyl-1-octen-6-inyl]-5-hydroxy-5,6-dihydro-4H-cyclopenta [b] furan-2-yl}-pentansäure.

19. 5-{(4R,5R)-4-[3S,4RS)-(E)-3-Hydroxy-4-methyl-1-nonen-7-inyl]-5-hydroxy-5,6-dihydro-4H-cyclopenta [b] furan-2-yl}-pentansäure.

20. 5-{(4R,5R)-4-[(3S)-(E)-3-Hydroxy-1-octenyl]-5-hydroxy-5,6-dihydro-4H-cyclopenta [b] furan-2-yl}-pentansäure-tris-(hydroxymethyl)-aminomethansalz.

21. 5-{(4R,5R)-4-[(3S,4RS)-(E)-3-Hydroxy-4-methyl-1-octenyl]-5-hydroxy-5,6-dihydro-4H-cyclopenta-[b] furan-2-yl}-pentansäuremethylsulfonylamid.

22. 5-{(4R,5R)-4-[(3S,4RS)-(E)-3-Hydroxy-4-methyl-1-octen-6-inyl]-5,6-dihydro-4H-cyclopenta [b] furan-2-yl}-pentansäure-Kaliumsalz.

23. Arzneimittel, bestehend aus einer oder mehreren Verbindungen gemäß Anspruch 1 und üblichen Hilfs- und Trägerstoffen.

24. Verfahren zur Herstellung von Prostacyclinen der allgemeinen Formel I, dadurch gekennzeichnet, daß man eine Verbindung der Formel II

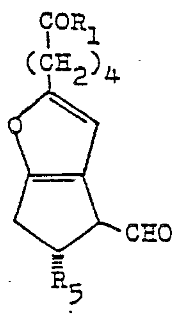

(II)

worin $R_1$ und $R_5$ die oben angegebene Bedeutungen aufweisen mit einem Alkaliphosphonat der allgemeinen Formel III

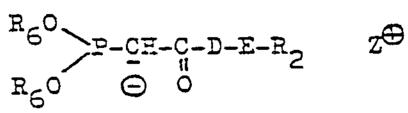

(III)

worin D, E und $R_2$ die oben angegebenen Bedeutungen haben und $R_6$ eine $C_1$-$C_4$-Alkyl und Z die Alkalimetalle Li, Na oder K bedeuten, umsetzt und je nach der gewünschten Bedeutung des Endprodukts reduziert oder mit Methylmagnesiumbromid oder mit Methyl-lithium umsetzt oder gegebenenfalls in die Stereoisomeren trennt oder den 1-Ester verseift oder die 1-Säure verestert oder mit einem isocyanat der Formel $R_4$—NCO umsetzt, worin $R_4$ die angegebene Bedeutung hat, oder OH-Schutzgruppen abspaltet.

25. Prostacyclinzwischenprodukte der allgemeinen Formel

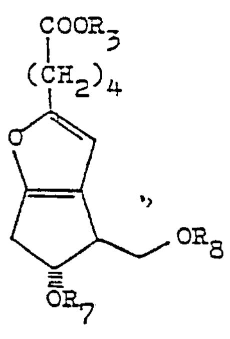

(VI)

worin $R_3$ die oben angegebene Bedeutung hat und $R_7$ und $R_8$ die für $R_5$ angegebenen Schutzgruppen, eine Tri-($C_1$-$C_4$)-alkylsilylgruppe oder Benzylgruppe bedeuten.

26. Verfahren zur Herstellung von Verbindungen der Formel VI

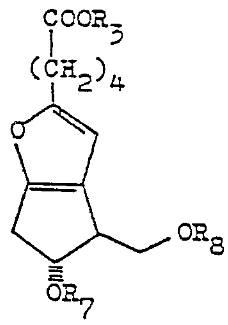

(VI),

worin $R_3$, $R_7$ und $R_8$ die oben angegebenen Bedeutungen haben, dadurch gekennzeichnet, daß man Verbindungen der allgemeinen Formel VII

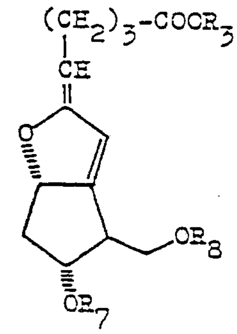

(VII),

mit $R_3$, $R_7$ und $R_8$ in den angegebenen Bedeutungen bei erhöhter Temperatur mit schwachen Lewissäuren behandelt.

**Claims**

1. Prostacyclin derivatives of the general formula I

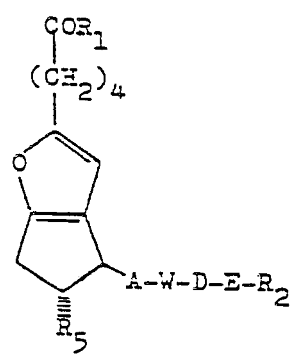

(I),

wherein

$R^1$ is the group $OR_3$, in which $R_3$ is hydrogen or alkyl of 1 to 10 carbon atoms (optionally substituted by halogen, aryl, $C_{1-4}$ alkoxy or $C_{1-4}$ dialkylamino), cycloalkyl, aryl or a heterocyclic group, or the group $NHR_4$, in which $R_4$ is hydrogen, alkanoyl or alkanesulphonyl each of 1-10 carbon atoms,

A is a $—CH_2—CH_2—$ or a trans $—CH=CH—$ group,

W is a 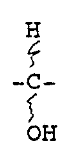 or a 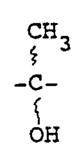 group,

in which the OH group can be respectively esterified by a benzoyl group or an alkanoyl group of 1-4 carbon atoms or etherified by a tetrahydropyranyl, tetrahydrofuranyl, $C_{1-4}$ alkoxyalkyl or tri($C_{1-4}$ alkyl)-

silyl group, and in which the free or esterified OH group can be in the α- or β-configuration,

D and E together form a direct bond or

D is a straight or branched alkylene group of 1 to 5 carbon atoms and

E is oxygen or a direct bond,

$R^2$ is a straight or branched alkyl group of 1-6 carbon atoms or a straight or branched alkenyl group of 2-6 carbon atoms that can be substituted by phenyl, halogen or $C_{1-4}$ alkyl and when D and E together form a direct bond, can also be an alkynyl group of 2 to 6 carbon atoms, optionally substituted in the 1-position by halogen or $C_{1-4}$ alkyl, or, when $R^1$ is a hydroxy group $R^5$ is a hydroxy group, A is a trans —CH=CH— group,

W is 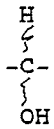 group

D is a $—CH_2—$ group and E is an O-atom, $R^2$ can also be phenyl,

$R^5$ is a hydroxy group that can be esterified by an alkanoyl group of 1-4 carbon atoms or etherified by a tetrahydropyranyl, tetrahydrofuranyl, alkoxyalkyl or trialkylsilyl group, and when $R_3$ is hydrogen, salts of these with physiologically acceptable bases.

2. 5-(4R,5R)-4-[(3S)-(E)-3-Hydroxy-1-octenyl]-5-(tetrahydropyran-2-yloxy)-5,6-dihydro-4H-cyclopenta [b] furan-2-yl-pentanoic acid, methyl ester.

3. 5-(4R,5R)-4-[(3R)-(E)-3-Hydroxy-1-octenyl]-5-(tetrahydropyran-2-yloxy)-5,6-dihydro-4H-cyclopenta [b] furan-2-yl-pentanoic acid, methyl ester.

4. 5-{(4R,5R)-4-[(3S)-(E)-3-Hydroxy-1-octenyl]-5-hydroxy-5,6-dihydro-4H-cyclopenta [b] furan-2-yl}-pentanoic acid, methyl ester.

5. 5-{(4R,5R)-4-[(3R)-(E)-3-Hydroxy-1-octenyl-5-hydroxy-5,6-dihydro-4H-cyclopenta [b] furan-2-yl}-pentanoic acid, methyl ester.

6. 5-{(4R,5R)-4-[(3S)-(E)-3-Hydroxy-1-octenyl]-5-hydroxy-5,6-dihydro-4H-cyclopenta [b] furan-2-yl}-pentanoic acid.

7. 5-{(4R,5R)-4-[(3R)-(E)-3-Hydroxy-1-octenyl]-5-hydroxy-5,6-dihydro-4H-cyclopenta [b] furan-2-yl}-pentanoic acid.

8. 5-{(4R,5R)-4-[(3S,4RS)-(E)-3-Hydroxy-4-methyl-1-octenyl]-5-hydroxy-5,6-dihydro-4H-cyclopenta [b] furan-2-yl}-pentanoic acid.

9. 5-{(4R,5R)-4-[(3R,4RS)-(E)-3-Hydroxy-4-fluoro-1-octenyl]-5-hydroxy-5,6-dihydro-4H-cyclopenta [b] furan-2-yl}-pentanoic acid.

10. 5-{(4R,5R)-4-[(3R)-(E)-3-Hydroxy-4-phenoxy-1-butenyl]-5-hydroxy-5,6-dihydro-4H-cyclopenta [b] furan-2-yl}-pentanoic acid.

11. 5-{(4R,5R)-4-[(3S,4RS)-(1E,6Z)-3-Hydroxy-4-methyl-7-chloro-1,6-octadienyl]-5-hydroxy-5,6-dihydro-4H-cyclopenta [b] furan-2-yl}-pentanoic acid.

12. 5-{(4R,5R)-4-[(3S,4RS)-(E)-3-Hydroxy-4,7-dimethyl-1,6-octadienyl]-5-hydroxy-5,6-dihydro-4H-cyclopenta [b] furan-2-yl}-pentanoic acid.

13. 5-{(4R,5R)-4-[(3S)-(1E,5Z)-3-Hydroxy-1,5-octadienyl]-5-(tetrahydropyran-2-yloxy)-5,6-dihydro-4H-cyclopenta [b] furan-2-yl}-pentanoic acid methyl ester.

14. 5-{(4R,5R)-4-[(3S)-(1E,5Z)-3-Hydroxy-1,5-octadienyl]-5-hydroxy-5,6-dihydro-4H-cyclopenta [b] furan-2-yl}-pentanoic acid.

15. 5-{(4R,5R)-4-[(3S,4RS)-(E)-3-Hydroxy-4-methyl-1-octen-6-inyl]-5-(tetrahydropyran-2-yloxy)-5,6-dihydro-4H-cyclopenta [b] furan-2-yl}-pentanoic acid, methyl ester.

16. 5-{(4R,5R)-4-[(3R,4RS)-(E)-3-Hydroxy-4-methyl-1-octen-6-inyl]-5-(tetrahydropyran-2-yloxy)-5,6-dihydro-4H-cyclopenta [b] furan-2-yl}-pentanoic acid, methyl ester.

17. 5-{(4R,5R)-4-[(3S,4RS)-(E)-3-Hydroxy-4-methyl-1-octen-6-inyl]-5-hydroxy-5,6-dihydro-4H-cyclopenta [b] furan-2-yl}-pentanoic acid.

18. 5-{(4R,5R)-4-[(3R,4RS)-(E)-3-Hydroxy-4-methyl-1-octen-6-inyl]-5-hydroxy-5,6-dihydro-4H-cyclopenta [b] furan-2-yl}-pentanoic acid.

19. 5-{(4R,5R)-4-[(3S,4RS)-(E)-3-Hydroxy-4-methyl-1-nonen-7-inyl]-5-hydroxy-5,6-dihydro-4H-cyclopenta [b] furan-2-yl}-pentanoic acid.

20. 5-{(4R,5R)-4-[(3S)-(E)-3-Hydroxy-1-octenyl]-5-hydroxy-5,6-dihydro-4H-cyclopenta [b] furan-2-yl}-pentanoic acid, tris(hydroxymethyl) aminomethane salt.

21. N-(Methylsulphonyl)-5-{(4R,5R)-4-[(3S,4RS)-(E)-3-hydroxy-4-methyl-1-octenyl]-5-hydroxy-5,6-dihydro-4H-cyclopenta [b] furan-2-yl}-pentanamide.

22. 5-{(4R,5R)-4-[(3S,4RS)-(E)-3-Hydroxy-4-methyl-1-octen-6-inyl]-5,6-dihydro-4H-cyclopenta [b] furan-2-yl}-pentanoic acid, potassium salt.

23. Pharmaceutical compositions comprising one or more compounds according to claim 1 and conventional additives and carriers.

24. Process for the preparation of prostacyclins of the general formula I, characterised in that a compound of formula II

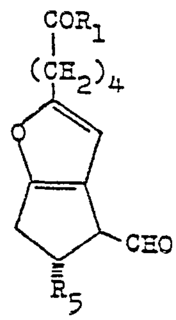

(II),

in which $R_1$ and $R_5$ have the meanings given above, is reacted with an alkali metal phosphonate of general formula III

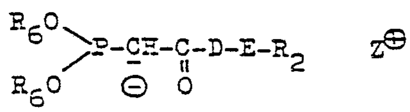

(III),

in which D, E and $R_2$ have the meanings given above, $R_6$ is a $C_{1-4}$ alkyl and Z is the alkali metal Li, Na or K and depending on the end products desired the compound is reduced, or reacted with methyl magnesium bromide or lithium methyl, or optionally separated into its stereoisomers, or the 1-ester is saponified, or the 1-acid is esterified or treated with an isocyanate of formula $R^4$—NCO, wherein $R^4$ has the meaning given above, or OH-protecting groups are split off.

25. Prostacyclin intermediates of the general formula VI

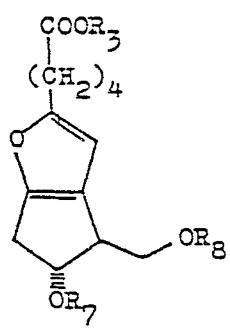

(VI),

in which $R_3$ has the meaning given above and $R_7$ and $R_8$ are the previously given protecting groups for $R_5$, a tri ($C_{1-4}$) alkylsilyl group or a benzyl group.

26. Process for the preparation of compounds of formula VI

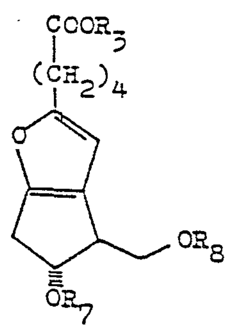

(VI),

in which $R_3$, $R_7$ and $R_8$ have the meanings given above, characterised in that a compound of general formula VII

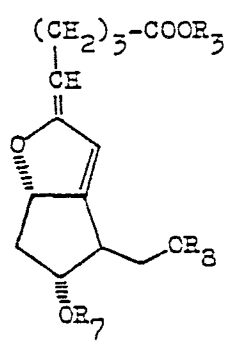

(VII),

in which $R_3$, $R_7$ and $R_8$ have the meanings given above is treated, at elevated temperature, with a weak Lewis acid.

**Revendications**

1. Prostacyclines répondant à la formule générale (I) :

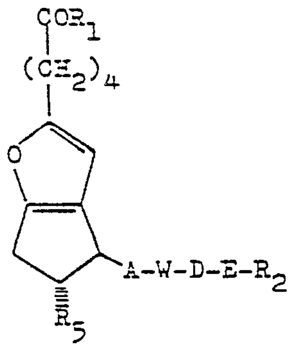

(I)

dans laquelle :

$R_1$ représente soit un radical $OR_3$ dans lequel $R_3$ désigne l'hydrogène, un alkyle en $C_1$-$C_{10}$ éventuellement porteur d'un halogène, d'un aryle, d'un alcoxy en $C_1$-$C_4$ ou d'un di-($C_1$-$C_4$) alkyl-amino, un cycloalkyle, un aryle ou un radical hétérocyclique, soit un radical $NHR_4$ dans lequel $R_4$ désigne un hydrogène, un alcanoyle en $C_1$-$C_{10}$ ou un alcane-sulfonyle en $C_1$-$C_{10}$,

A représente un radical $—CH_2—CH_2—$ ou un radical $—CH=CH—$ trans,

W représente un radical 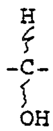 ou un radical 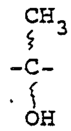

dans chacun desquels le radical OH peut être estérifié par un radical benzoyle ou par un radical alcanoyle en $C_1$-$C_4$ ou être ethérifié par un radical tétrahydropyrannyle, tétrahydrofurannyle, ($C_1$-$C_4$) alcoxy-alkyle ou tris-($C_1$-$C_4$-alkyl)-silyle, le radical hydroxy, qu'il soit libre ou estérifié, pouvant avoir la configuration $\alpha$ ou la configuration $\beta$,

D et E forment ensemble une liaison directe ou

D représente un radical alkylène en $C_1$-$C_5$, linéaire ou ramifié, et

E un atome d'oxygène ou une liaison directe,

$R_2$ représente un radical alkyle en $C_1$-$C_6$, linéaire ou ramifié, un radical alcényle en $C_2$-$C_6$, linéaire ou ramifié, éventuellement porteur d'un phényle, d'un halogène ou d'un alkyle en $C_1$-$C_4$, ou — dans le cas où D et E forment ensemble une liaison directe — un radical alcynyle qui contient de 2 à 6 atomes de carbone et qui porte éventuellement, en position 1, un halogène ou un alkyle en $C_1$-$C_4$, ou encore dans le cas où $R_1$ est un radical hydroxy, $R_5$ un radical hydroxy, A un radical $—CH=CH—$, W un radical

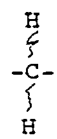

O un radical $—CH_2—$ et O un atome d'Oxygène O, un radical phényle, et

$R_5$ représente un radical hydroxy qui peut être estérifié par un radical d'acide alcanoïque en $C_1$-$C_4$ ou éthérifié par un radical tétrahydropyrannyle, tétrahydrofurannyle, alcoxyalkyle ou trialkyl-silyle, et, dans le cas où $R_3$ représente un atome d'hydrogène, sels que forment ces composés avec des bases acceptables du point de vue physiologique.

2. {[Hydroxy-3 octène-1 yl-(3S)-(E)]-4 (tétrahydropyrannyl-2 oxy)-5 dihydro-5,6 4H-cyclopenta [b] furannyl-2 (4R,5R)}-5 pentanoate de méthyle.

3. {[Hydroxy-3 octène-1 yl-(3R)-(E)]-4 (tétrahydropyrannyl-2 oxy)-5 dihydro-5,6 4H-cyclopenta [b] furannyl-2 (4R,5R)}-5 pentanoate de méthyle.

4. {[Hydroxy-3 octène-1 yl-(3S)-(E)]-4 hydroxy-5 dihydro-5,6 4H-cyclopenta [b] furannyl-2 (4R,5R)}-5 pentanoate de méthyle.

5. {[Hydroxy-3 octène-1 yl-(3R)-(E)]-4 hydroxy-5 dihydro-5,6 4H-cyclopenta [b] furannyl-2 (4R,5R)}-5 pentanoate de méthyle.

6. Acide {[hydroxy-3 octène-1 yl-(3S)-(E)]-4 hydroxy-5 dihydro-5,6 4H-cyclopenta [b] furannyl-2 (4R,5R)}-5 pentanoïque.

7. Acide {[hydroxy-3 octène-1 yl-(3R)-(E)]-4 hydroxy-5 dihydro-5,6 4H-cyclopenta [b] furannyl-2 (4R,5R)}-5 pentanoïque.

8. Acide {[hydroxy-3 méthyl-4 octène-1 yl-(3S,4RS)-(E)]-4 hydroxy-5 dihydro-5,6 4H-cyclopenta [b] furannyl-2 (4R,5R)}-5 pentanoïque.

9. Acide {[hydroxy-3 fluoro-4 octène-1 yl-(3R,4RS)-(E)]-4 hydroxy-5 dihydro-5,6 4H-cyclopenta [b] furannyl-2 (4R,5R)}-5 pentanoïque.

10. Acide {[hydroxy-3 phénoxy-4 butène-1 yl-(3R)-(E)]-4 hydroxy-5 dihydro-5,6 4H-cyclopenta [b] furannyl-2 (4R,5R)}-5 pentanoïque.

11. Acide {[hydroxy-3 méthyl-4 chloro-7 octadiène-1,6 yl-(3S,4RS)-(1E,6Z)]-4 hydroxy-5 dihydro-5,6 4H-cyclopenta [b] furannyl-2 (4R,5R)}-5 pentanoïque.

12. Acide {[hydroxy-3 diméthyl-4,7 octadiène-1,6 yl-(3S,4RS)-(E)]-4 hydroxy-5 dihydro-5,6 4H-cyclopenta [b] furannyl-2 (4R,5R)}-5 pentanoïque.

13. {[Hydroxy-3 octadiène-1,5 yl-(3S)-(1E,5Z)]-4 (tétrahydropyrannyl-2 oxy)-5 dihydro-5,6 4H-cyclopenta [b] furannyl-2 (4R,5R)}-5 pentanoate de méthyle.

14. Acide {[hydroxy-3 octadiène-1,5 yl-(3S)-(1E,5Z)]-4 hydroxy-5 dihydro-5,6 4H-cyclopenta [b] furannyl-2 (4R,5R)}-5 pentanoïque.

15. {[Hydroxy-3 méthyl-4 octène-1 yne-6 yl-(3S,4RS)-(E)]-4 (tétrahydropyrannyl-2 oxy)-5 dihydro-5,6 4H-cyclopenta [b] furannyl-2 (4R,5R)}-5 pentanoate de méthyle.

16. {[Hydroxy-3 méthyl-4 octène-1 yne-6 yl-(3R,4RS)-(E)]-4 (tétrahydropyrannyl-2 oxy)-5 dihydro-5,6 4H-cyclopenta [b] furannyl-2 (4R,5R)}-5 pentanoate de méthyle.

17. Acide {[hydroxy-3 méthyl-4 octène-1 yne-6 yl-(3S,4RS)-(E)]-4 hydroxy-5 dihydro-5,6 4H-cyclopenta [b] furannyl-2 (4R,5R)}-5 pentanoïque.

18. Acide {[hydroxy-3 méthyl-4 octène-1 yne-6 yl-(3R,4RS)-(E)]-4 hydroxy-5 dihydro-5,6 4H-cyclopenta [b] furannyl-2 (4R,5R)}-5 pentanoïque.

19. Acide {[hydroxy-3 méthyl-4 nonène-1 yne-7 yl-(3S,4RS)-(E)]-hydroxy-5 dihydro-5,6 4H-cyclopenta [b] furannyl-2 (4R,5R)}-5 pentanoïque.

20. Sel formé par l'acide {[hydroxy-3 octène-1 yl-(3S)-(E)]-4 hydroxy-5 dihydro-5,6 4H-cyclopenta [b] furannyl-2 (4R,5R)}-5 pentanoïque et le tris-(hydroxyméthyl)-aminométhane.

21. Méthylsulfonylamide de l'acide {[hydroxy-3 méthyl-4 octène-1 yl-(3S,4RS)-(E)]-4 hydroxy-5 dihydro-5,6 4H-cyclopenta [b] furannyl-2 (4R,5R)}-5 pentanoïque.

22. {[Hydroxy-3 méthyl-4 octène-1 yne-6 yl-(3S,4RS)-(E)]-4 hydroxy-5 dihydro-5,6 4H-cyclopenta [b] furannyl-2 (4R,5R)}-5 pentanoate de potassium.

23. Médicament qui contient un ou plusieurs composés selon la revendication 1 ainsi que des adjuvants et excipients usuels.

24. Procédé pour préparer des prostacyclines de formule générale (I), procédé caractérisé en ce qu'on fait réagir un composé répondant à la formule (II) :

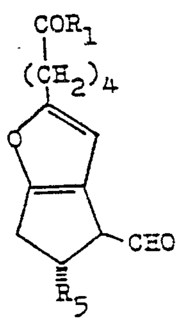

(II)

dans laquelle $R_1$ et $R_5$ ont les significations précédemment données, avec un phosphonate de métal alcalin répondant à la formule générale (III) :

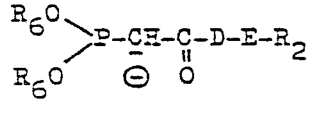

$Z^{\oplus}$ (III)

dans laquelle D, E et $R_2$ ont les significations précédemment données, $R_6$ représente un alkyle en $C_1$-$C_4$ et Z un métal alcalin, en l'espèce Li, Na ou K, et, selon les significations que doivent avoir les différents symboles dans le produit final, on réduit ou on fait réagir avec le bromure de méthyl-magnésium ou le méthyl-lithium ou on sépare éventuellement en stéréo-isomères ou on saponifie la fonction ester en position 1 ou on estérifie la fonction acide en position 1 ou on fait réagir avec un isocyanate $R_4$—NCO dans lequel $R_4$ a la signification précédemment donnée, ou on coupe des radicaux protecteurs de radicaux OH.

25. Corps intermédiaires pour prostacyclines, corps qui répondent à la formule générale (VI) :

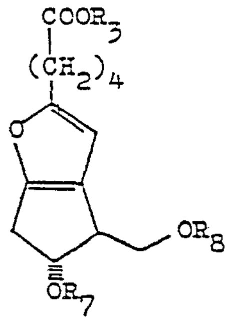

(VI)

dans laquelle $R_3$ a la signification précédemment donnée tandis que $R_7$ et $R_8$ représentent chacun l'un des radicaux protecteurs qui ont été indiqués pour $R_5$, un radical tris-($C_1$-$C_4$)alkyl-silyle ou un radical benzyle.

26. Procédé de préparation de composés répondant à la formule (VI) :

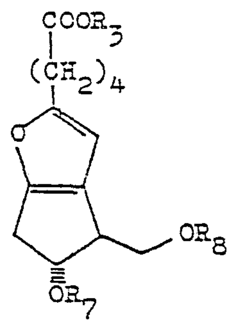

(VI)

dans laquelle $R_3$, $R_7$ et $R_8$ ont les significations précédemment données, procédé caractérisé en ce qu'on traite des composés répondant à la formule générale (VII) :

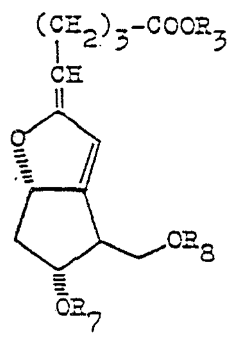

(VII)

dans laquelle $R_3$, $R_7$ et $R_8$ ont les significations précédemment données, à température élevée, par des acides de Lewis faibles.